# FASCICULE DE BREVET EUROPEEN

(11) **EP 0 970 686 B1**
(45) Date de publication et mention de la délivrance du brevet: **26.04.2006**
(21) Numéro de dépôt: 99401548.5
(22) Date de dépôt: 22.06.1999
(51) Int. Cl.: A61K 8/49, A61K 8/73, A61Q 5/10

(54) **Composition de teinture pour fibres kératiniques avec un colorant direct cationique et un polymère épaississant**
Färbemittel für Keratinfasern, das ein Kationischer Farbstoffe und eine Verdickendes Polymer enthält
Composition for dyeing keratinous fibres containing a cationic direct dye and a thickening polymer

(30) Priorité: 09.07.1998 FR 9808832
(43) Date de publication de la demande: 12.01.2000
(73) Titulaire: L'OREAL, 75008 Paris (FR)
(72) Inventeur: Lang, Gérard, 95390 Saint Prix (FR); Cotteret, Jean, 78480 Verneuil Sur Seine (FR)
(74) Mandataire: Miszputen, Laurent

(56) Documents cités:
- EP-A- 0 801 942
- FR-A- 2 140 205
- FR-A- 2 189 006
- FR-A- 2 282 860
- FR-A- 2 285 851
- GB-A- 2 142 348

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins un polymère épaississant particulier.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, Il arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes sur le plan de la puissance, de l'homogénéité de la couleur répartie le long de la fibre, on dit alors que la coloration est trop sélective, et sur le plan de la tenacité, en terme de résistance aux diverses agressions que peuvent subir les cheveux (lumière, intempéries,shampooings).

Or, après d'importantes recherches menées sur la question, la demanderesse vient maintenant de découvrir qu'il est possible d'obtenir de nouvelles compositions pour la teinture des fibres kératiniques capables de conduire à des colorations plus puissantes et néanmoins peu sélectives et résistant bien aux diverses agressions que peuvent subir les cheveux, en associant au moins un polymère épaississant particulier à au moins un colorant direct cationique connu de l'art antérieur et de formules respectivement définies ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, (i)au moins un colorant direct cationique dont la structure répond aux formules (I) à (III) définies ci-après, caractérisée par le fait qu'elle contient en outre (ii)au moins un polymère épaississant particulier.
(**i**) Le colorant direct cationique utilisable selon la présente invention est un composé de formule (I) suivante :

   **A―N=N―B** (I)

   dans laquelle :
   **le symbole A** représente un groupement choisi parmi les structures A₁ à A₃ suivantes : 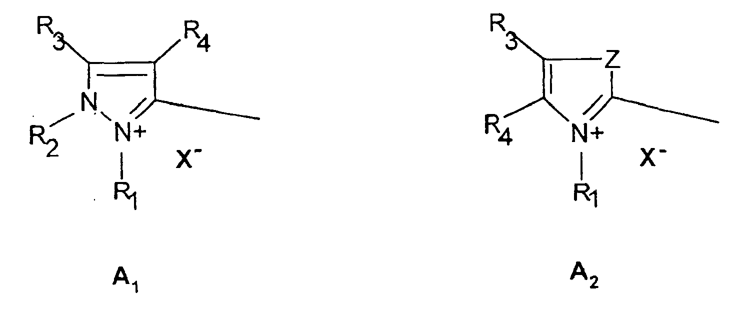 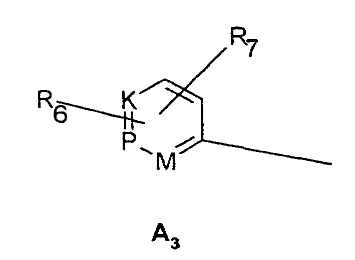 structures A₁ à A₃ dans lesquelles,
      R₁ désigne un radical alkyle en C₁-C₄, un radical phényle pouvant être substitué par un radical alkyle en C₁-C₄ ou un atome d'halogène choisi parmi le chlore, le brome, l'iode et le fluor ;
      R₂ désigne un radical alkyle en C₁-C₄ ou un radical phényle;
      R₃ et R₄, identiques ou différents, représentent un radical alkyle en C₁-C₄ , un radical phényle, ou bien, dans le cas de la structure A1, peuvent former ensemble un cycle benzénique substitué par un ou plusieurs radicaux alkyle en C₁-C₄ , alcoxy en C₁-C₄, ou NO₂, et dans le cas de la structure A2, peuvent former ensemble un cycle benzénique éventuellement substitué par un ou plusieurs radicaux alkyle en C₁-C₄ , alcoxy en C₁-C₄, ou NO₂;
      R₃ peut en outre désigner un atome d'hydrogène;
      Z désigne un atome d'oxygène, de soufre ou un groupement -NR₂;
      M représente un groupement -CH, -CR (R désignant alkyle en C₁-C₄), ou -NR₅(X⁻)ᵣ;
      K représente un groupement -CH, -CR (R désignant alkyle en C₁-C₄), ou -NR₅(X⁻)ᵣ;
      P représente un groupement -CH, -CR (R désignant alkyle en C₁-C₄), ou -NR₅(X⁻)ᵣ; r désigne zéro ou 1;
      R₅ représente un atome O⁻, un radical alcoxy en C₁-C₄, ou un radical alkyle en C₁-C₄;
      R₆ et R₇, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
      X⁻ représente un anion de préférence choisi parmi le chlorure, l'iodure, le méthyl sulfate, l'éthyl sulfate, l'acétate et le perchlorate;
      sous réserve que,
      si R₄ désigne un radical alkyle en C₁-C₄ et Z désigne un atome de soufre, R₃ ne désigne pas un atome d'hydrogène;
      si R₅ désigne O⁻, alors r désigne zéro;
      si K ou P ou M désignent -N-alkyle C₁-C₄ X⁻ , alors R₆ ou R₇ est différent d'un atome d'hydrogène;
      si K désigne -NR₅(X⁻)ᵣ, alors M= P= -CH; -CR;
      si M désigne -NR₅(X⁻)ᵣ, alors K= P= -CH; -CR;
      si P désigne -NR₅(X⁻)ᵣ, alors K= M et désignent -CH ou -CR;
      si Z désigne -NR₂ et R₂ désigne un radical alkyle en C₁-C₄ , alors au moins un des radicaux R₁, R₃ ou R₄ du groupement de structure **A**_{**2**} est différent d'un radical alkyle en C₁-C₄;
   **le symbole B** représente :
      - **(a)** un groupement de structure B₁ suivante : 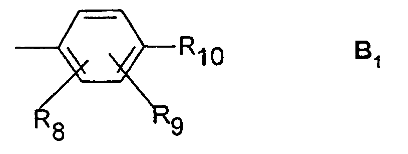 structure B₁ dans laquelle,
         R₈ représente un atome d'hydrogène, un atome d'halogène choisi parmi le chlore, le brome, l'iode et le fluor, un radical alkyle en C₁-C₄, alcoxy en C₁-C₄, un radical - OH, -NO₂, -NHR₁₁, -NR₁₂R₁₃, -NHCOalkyle en C₁-C₄, ou forme avec R₉ un cycle à 5 ou 6 chaînons contenant ou non un ou plusieurs hétéroatomes choisis parmi l'azote, l'oxygène ou le soufre;
         R₉ représente un atome d'hydrogène, un atome d'halogène choisi parmi le chlore, le brome, l'iode et le fluor, un radical alkyle en C₁-C₄, alcoxy en C₁-C₄,
      ou forme avec R₁₀ ou R₁₁ un cycle à 5 ou 6 chaînons contenant ou non un ou plusieurs hétéroatomes choisis parmi l'azote, l'oxygène ou le soufre;
      R₁₀ représente un atome d'hydrogène, un radical -OH, un radical -NHR₁₁, un radical -NR₁₂R₁₃;
      R₁₁ représente un atome d'hydrogène, un radical alkyle en C₁-C₄, un radical monohydroxyalkyle en C₁-C₄, polyhydroxyalkyle en C₂-C₄, un radical phényle;
      R₁₂ et R₁₃, identiques ou différents, représentent un radical alkyle en C₁-C₄ , un radical monohydroxyalkyle en C₁-C₄, polyhydroxyalkyle en C₂-C₄;
      - **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure B2 suivante : 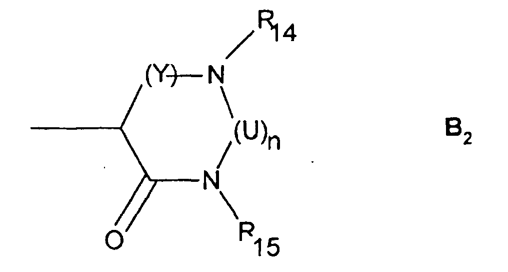 structure B₂ dans laquelle,
         R₁₄ et R₁₅, identiques ou différents, représentent un atome d'hydrogène , un radical alkyle en C₁-C₄, un radical phényle;
         Y désigne le radical -CO- ou le radical 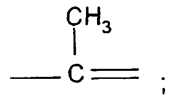 n = 0 ou 1, avec, lorsque n désigne 1, U désigne le radical -CO- .

      Dans les structures définies ci-dessus le groupement alkyle ou alcoxy en C₁-C₄ désigne de préférence méthyle, éthyle, butyle, méthoxy, éthoxy.
      Les colorants directs cationiques de formule (I) utilisables dans les compositions tinctoriales conformes à l'invention, sont des composés connus et sont décrits par exemple dans les demandes de brevets FR 2282860, FR-2189006, FR-2285851et FR-2140205 et ses certificats d'addition.
      Parmi les colorants directs cationiques de formule (I) utilisables dans les compositions tinctoriales conformes à l'invention, on peut citer plus particulièrement les composés de structures (I)₁ à (I)₇₇ suivantes : 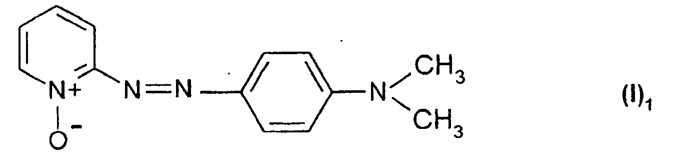 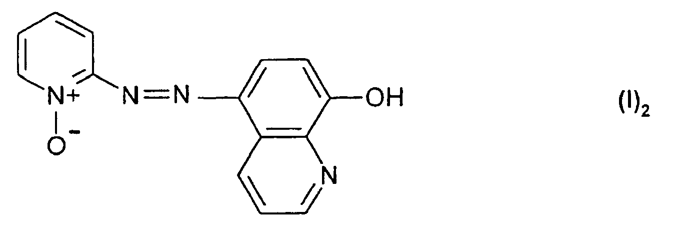 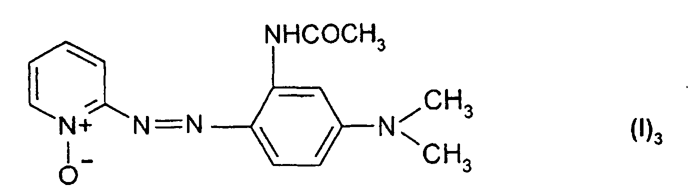 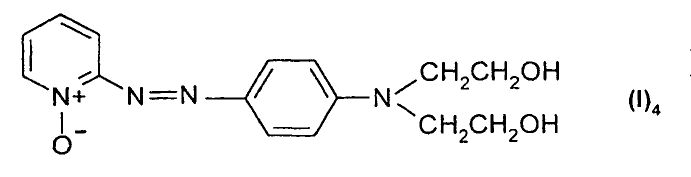 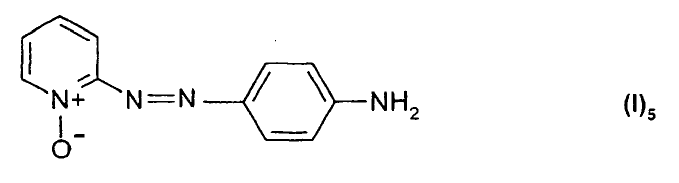 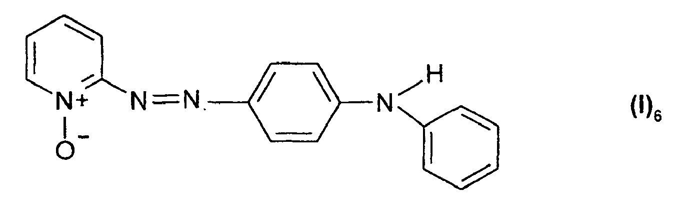 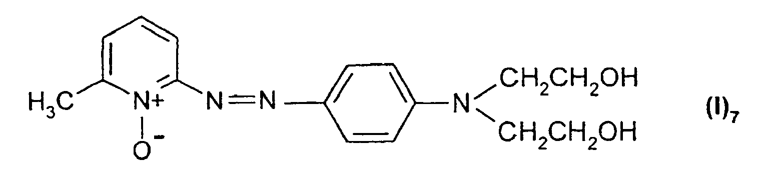 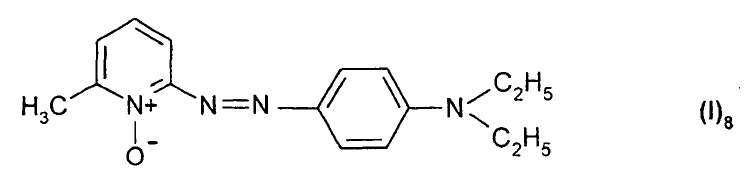 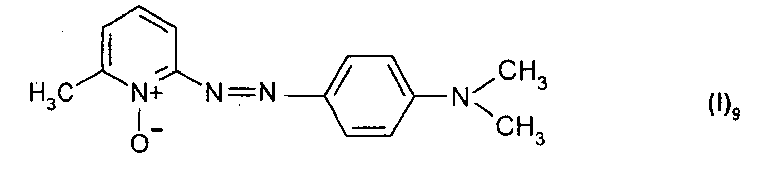 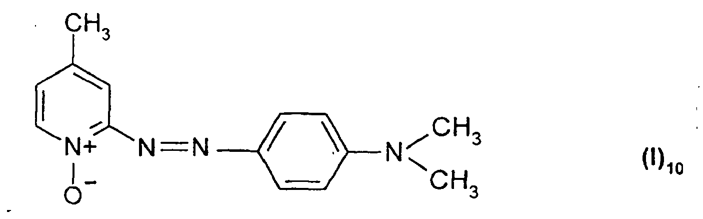 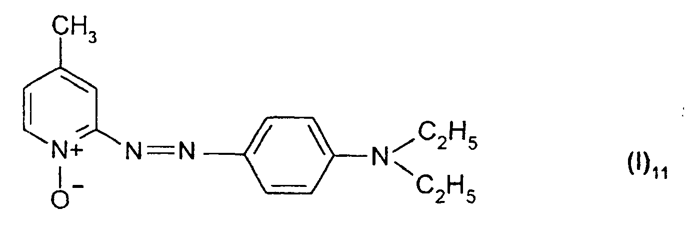 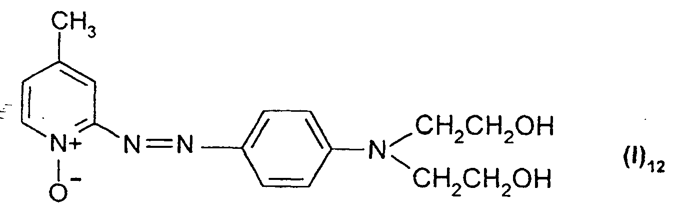 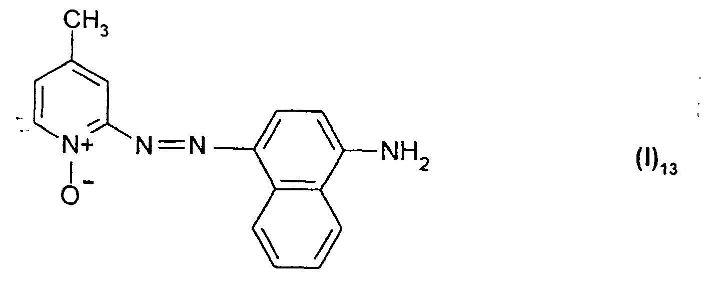 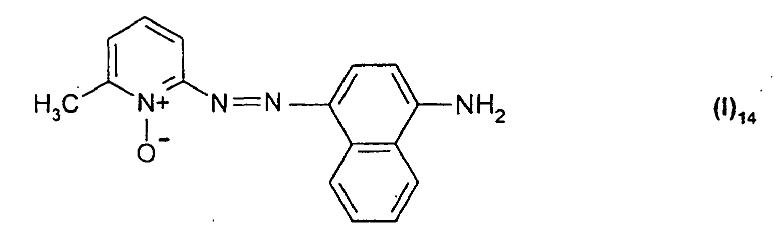 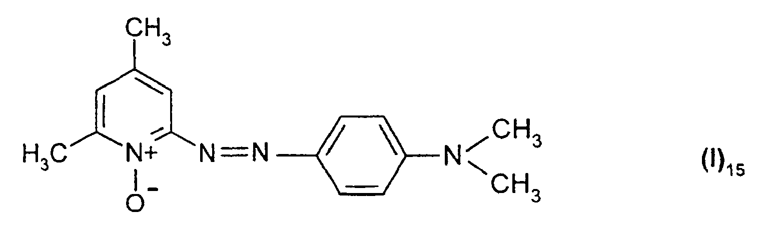 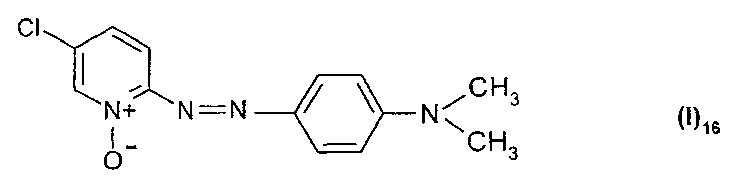 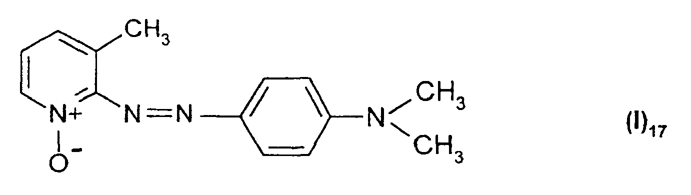 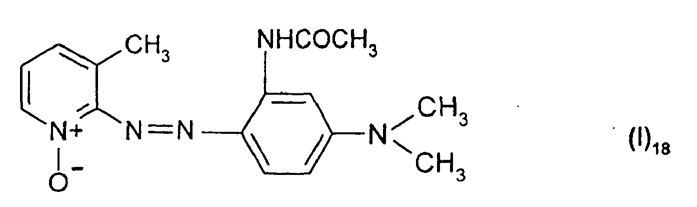 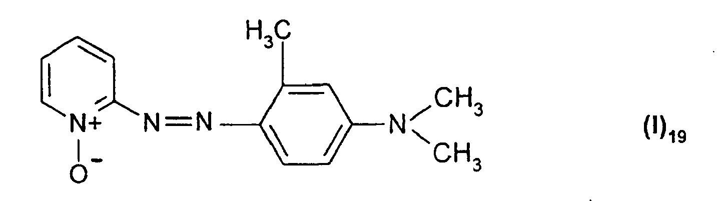 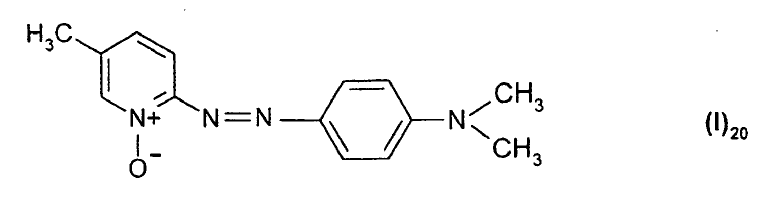 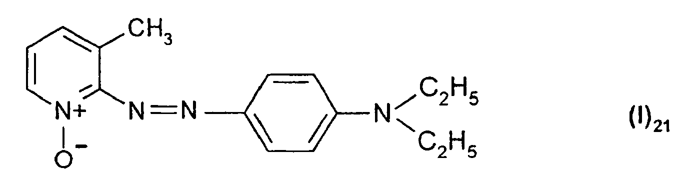 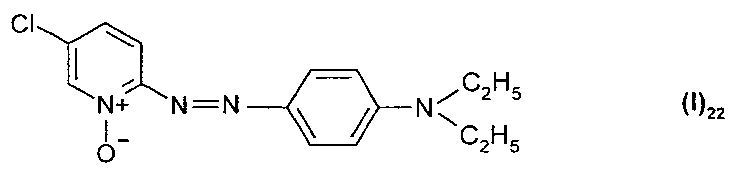 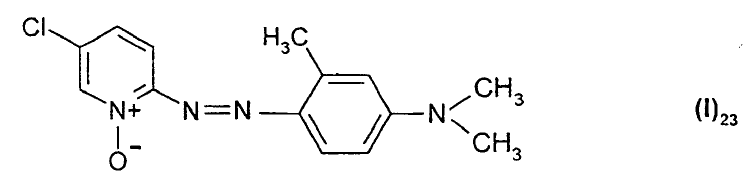 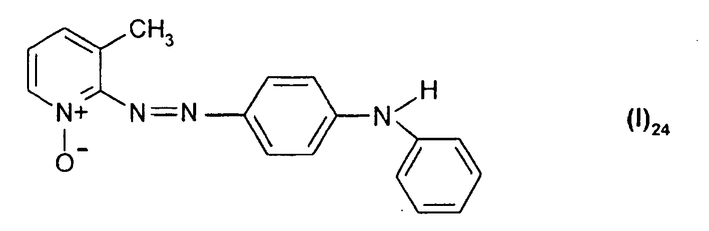 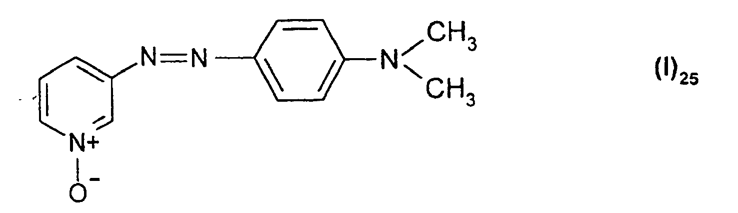 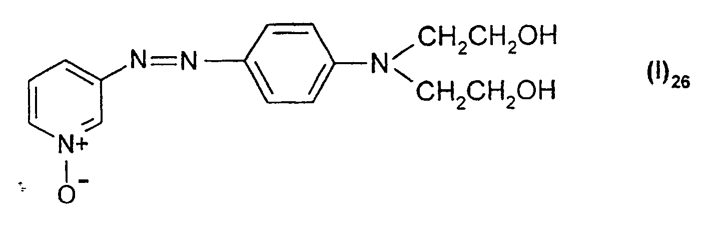 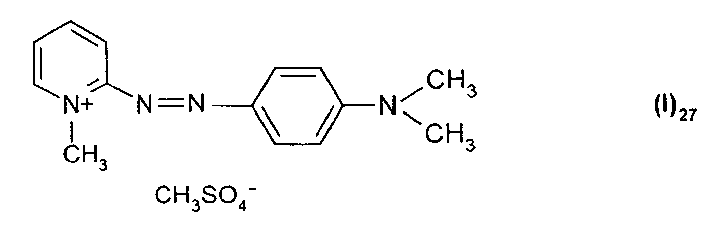 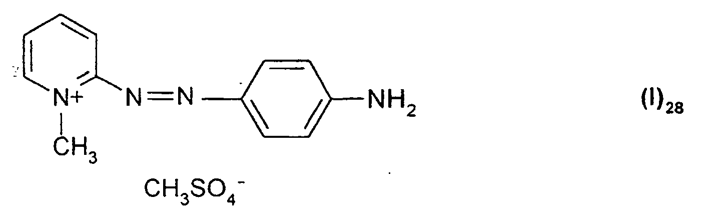 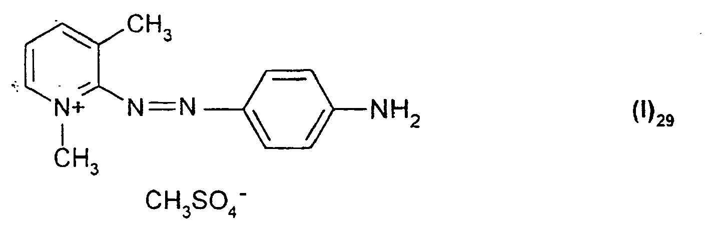 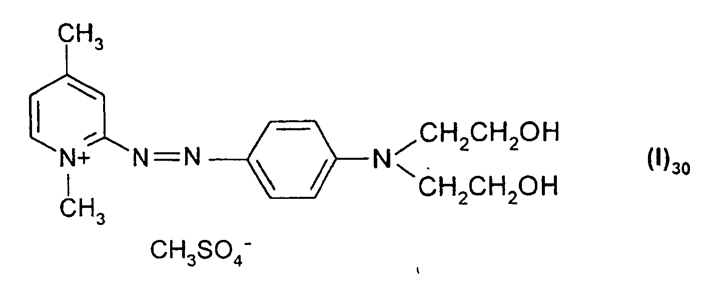 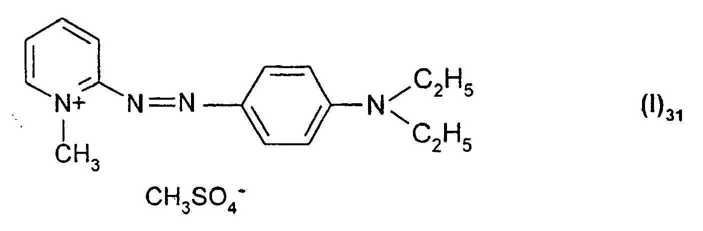 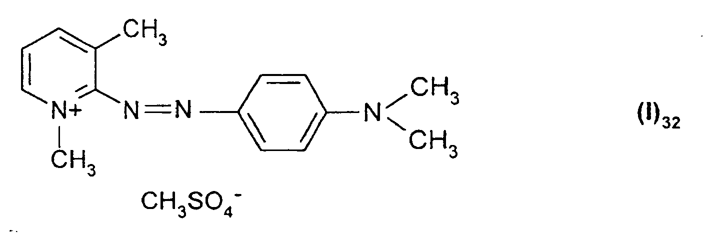 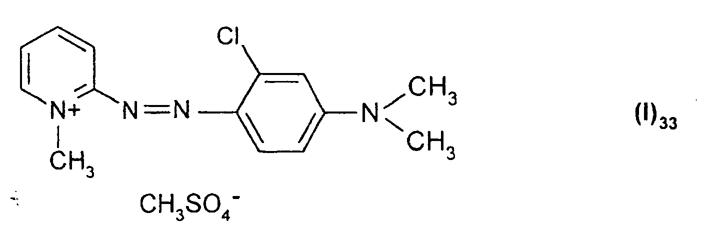 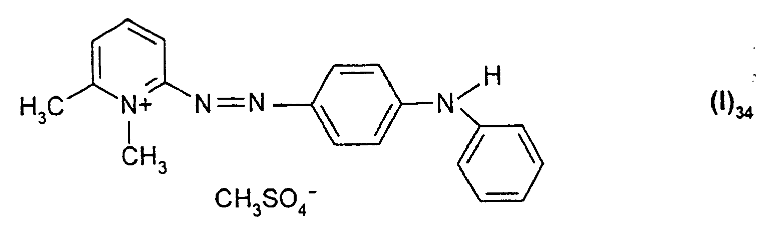 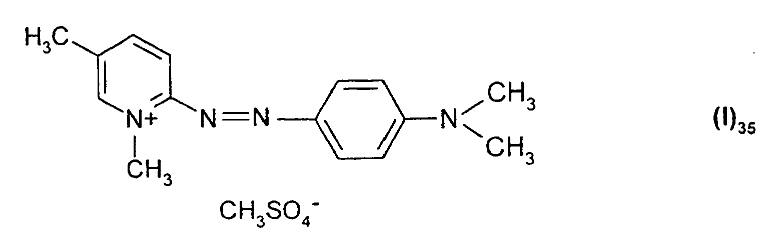 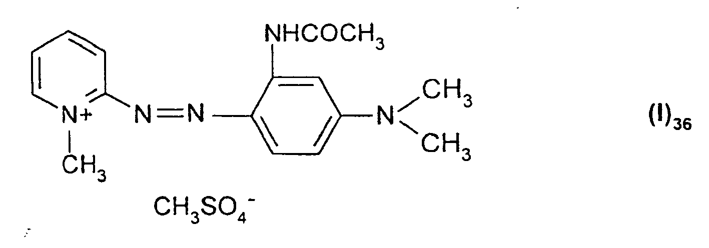 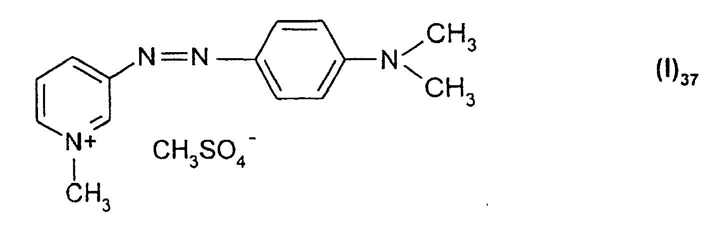 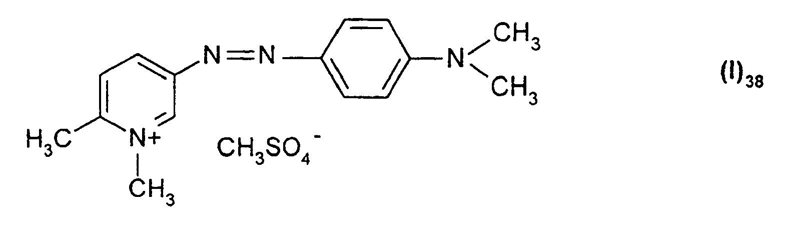 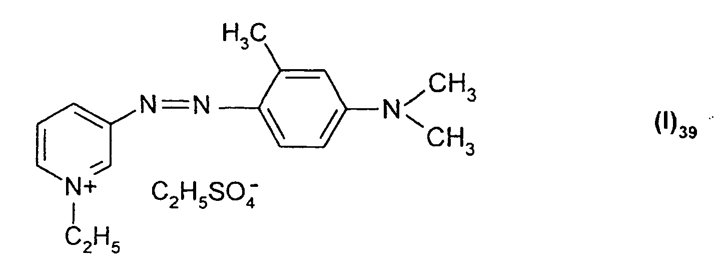 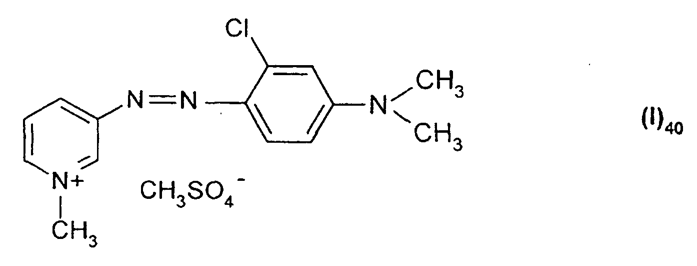 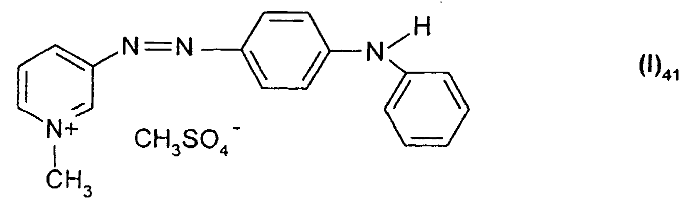 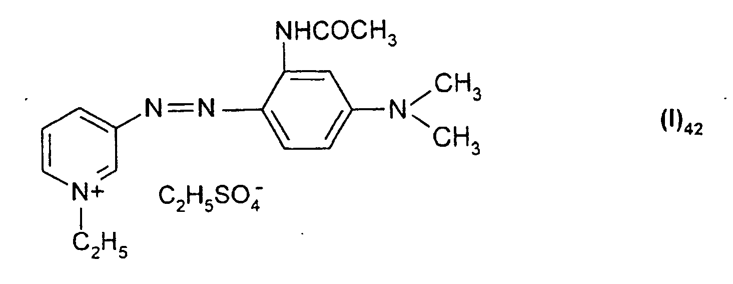 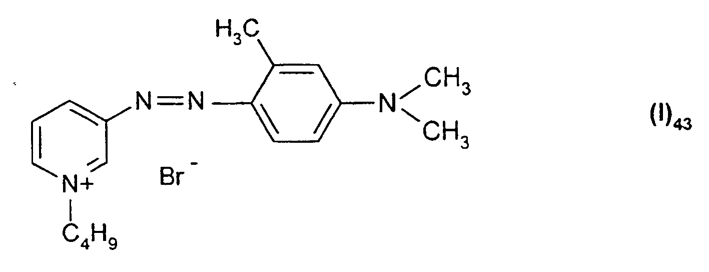 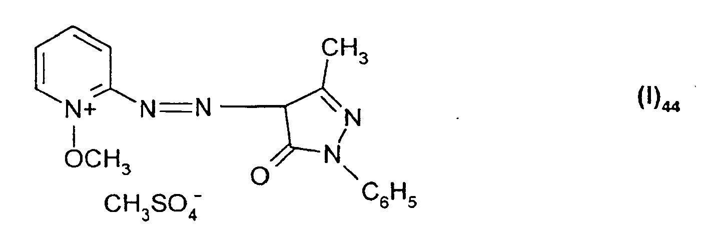 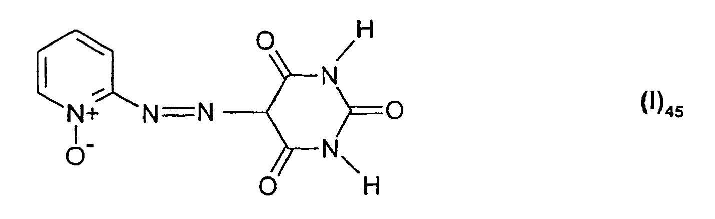 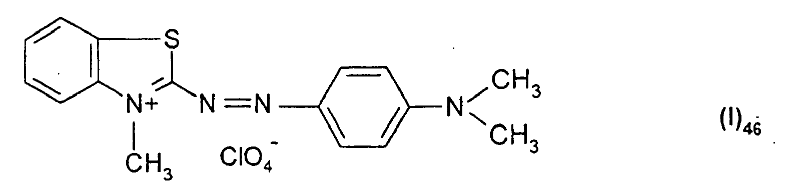 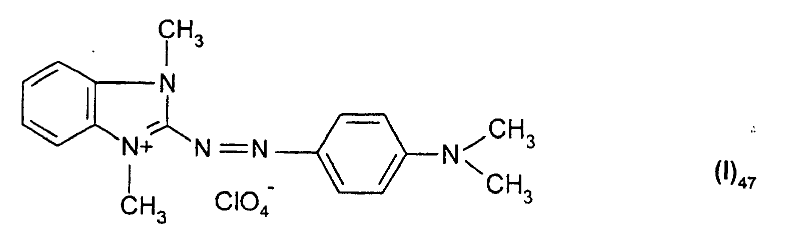 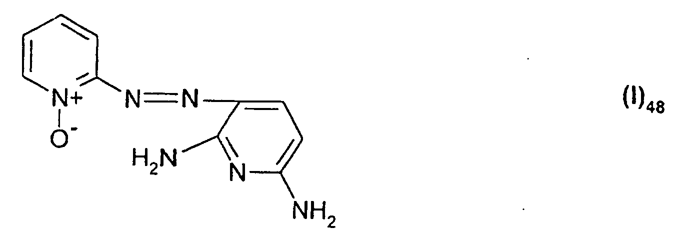 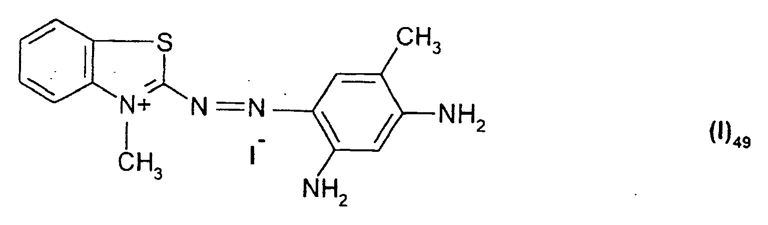 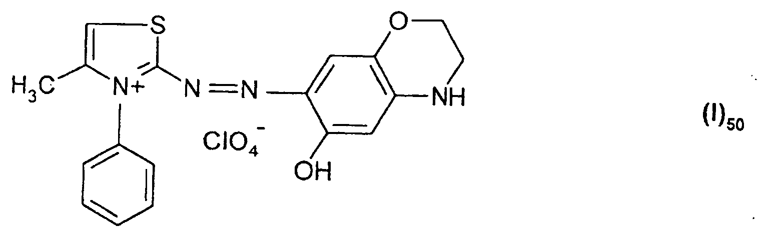 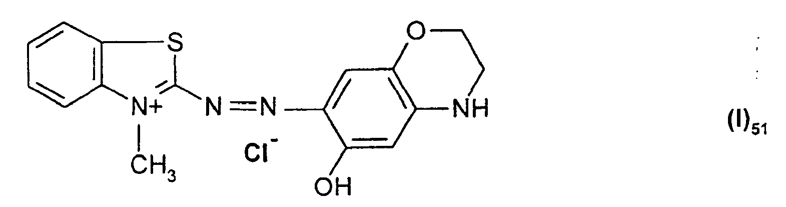 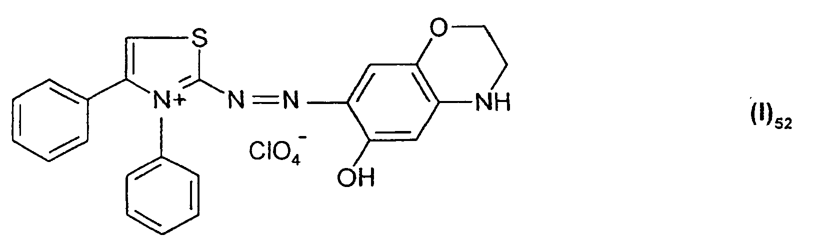 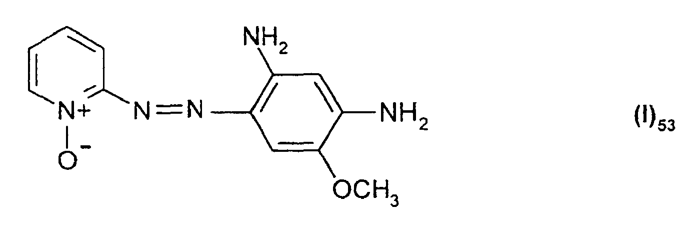 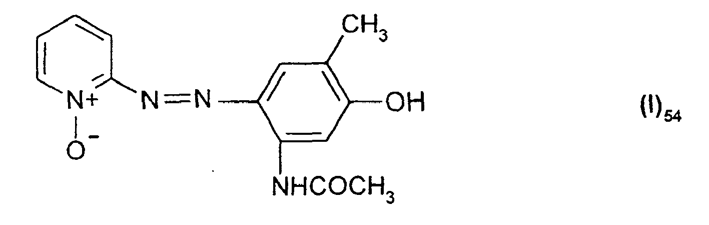 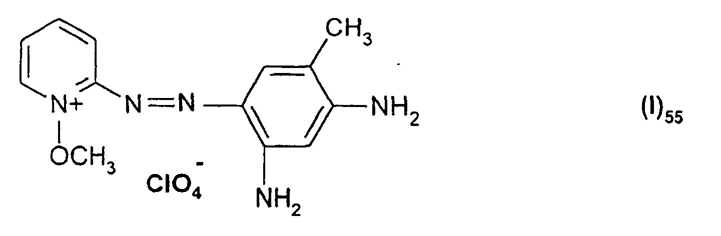 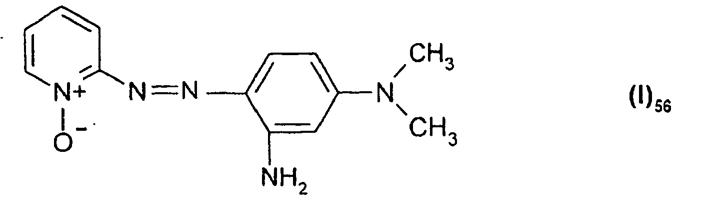 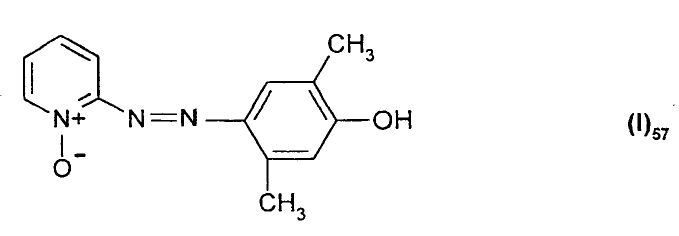 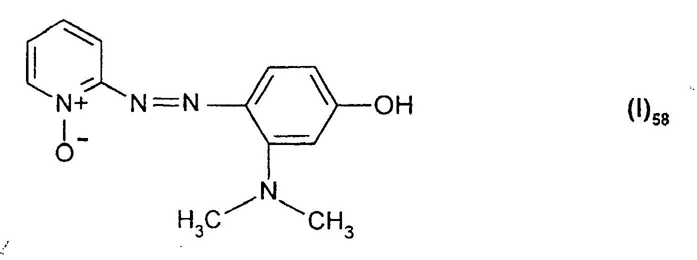 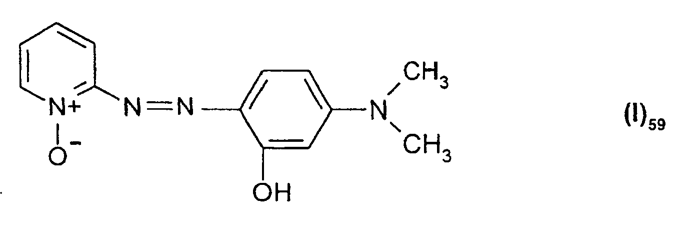 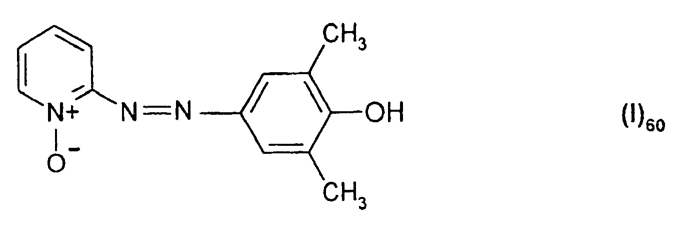 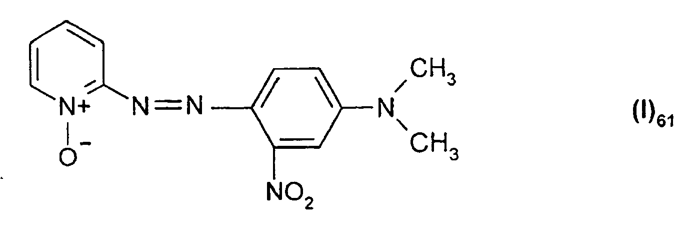 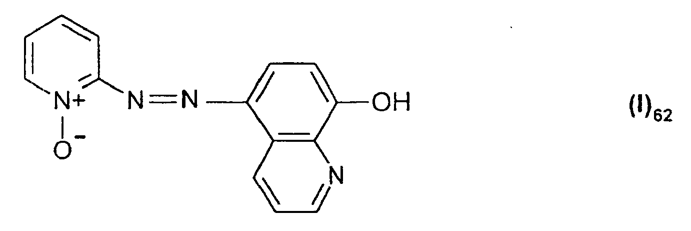 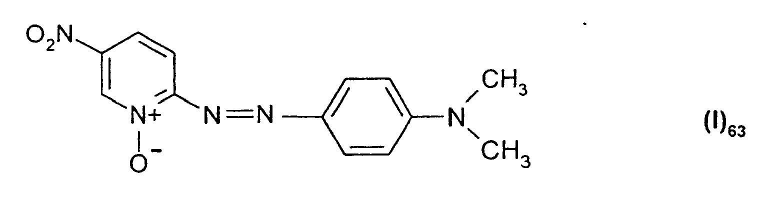 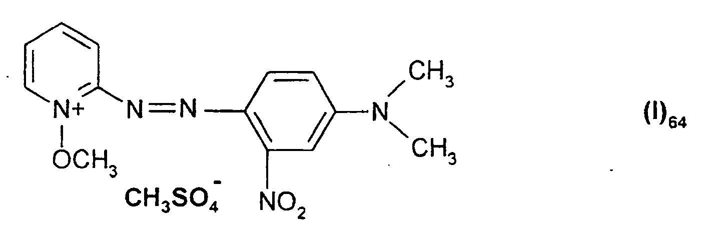 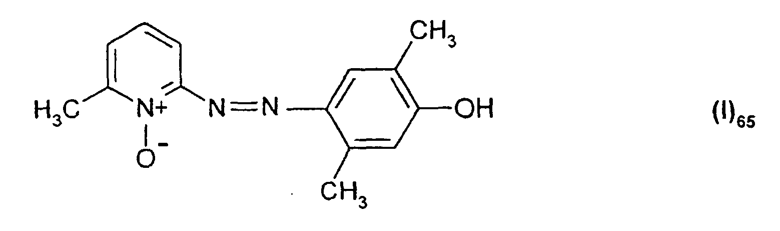 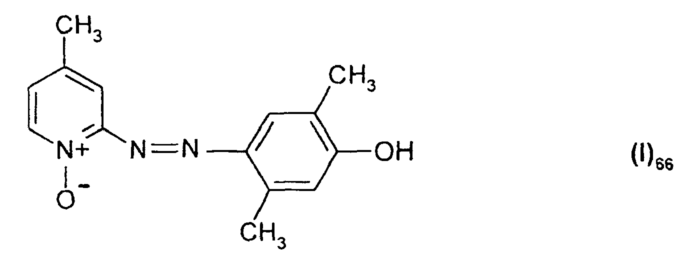 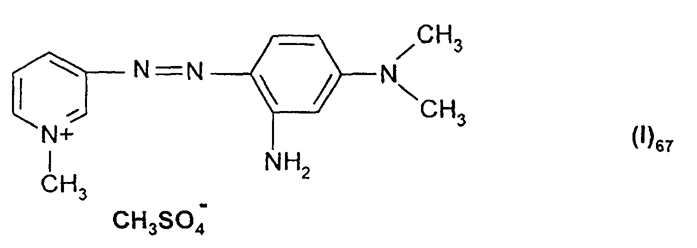 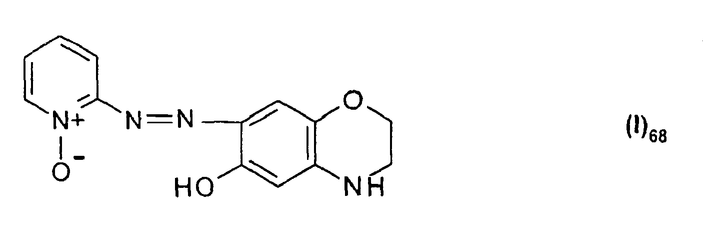 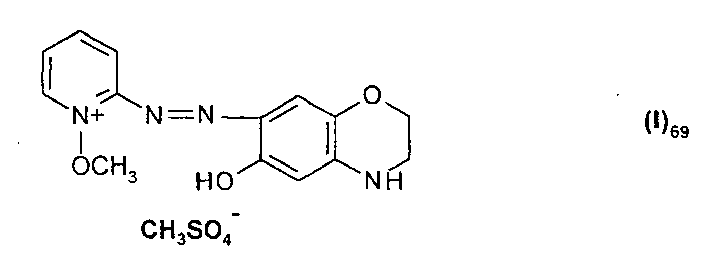 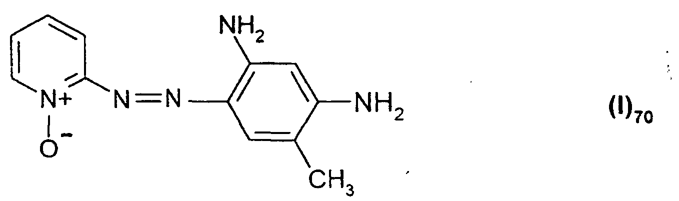 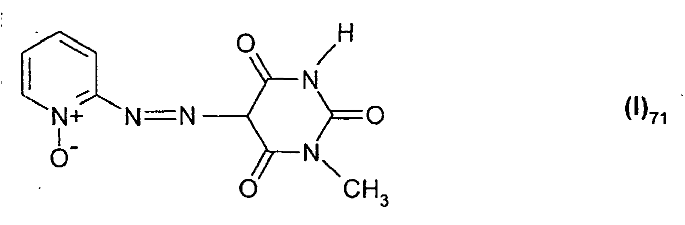 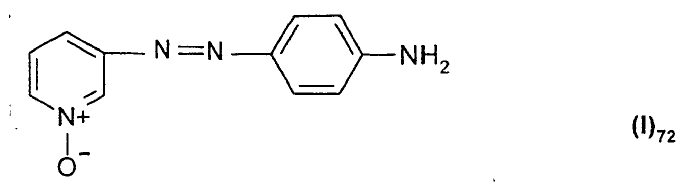 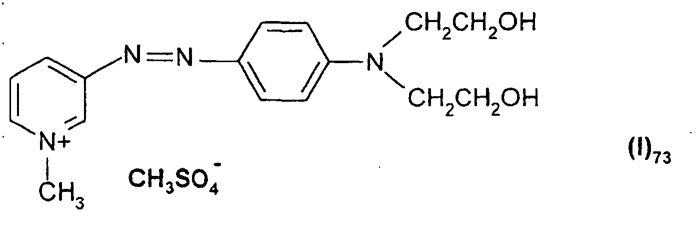 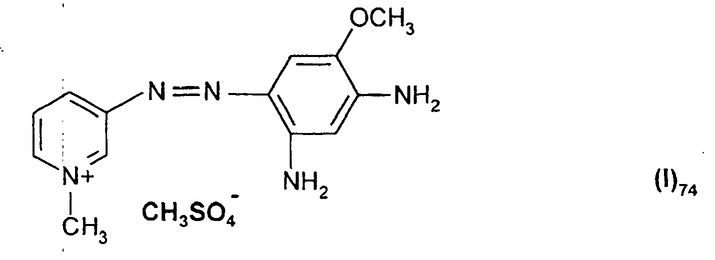 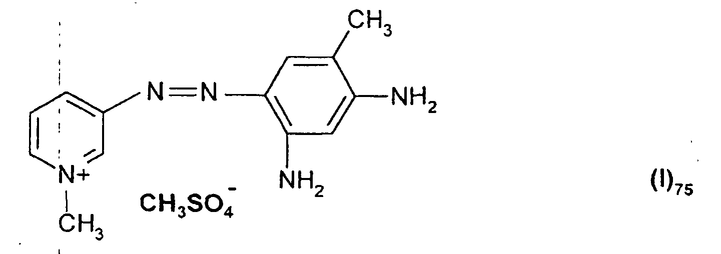 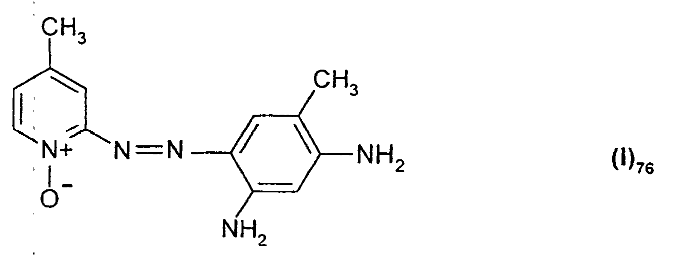 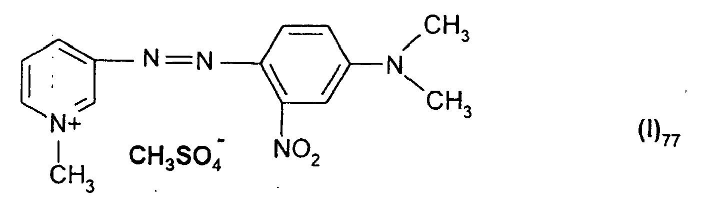
      Le ou les colorants directs cationiques de formule (I) utilisés selon l'invention, représentent de préférence de 0,001 à 10 % en poids environ du poids total de la composition tinctoriale et encore plus préférentiellement de 0,005 à 5 % en poids environ de ce poids.
**(ii)** Le polymère épaississant utilisable selon la présente invention est un polysaccharide ou une cellulose choisi dans le groupe constitué par :
   **(ii)**_{**1**} - les gommes de guar non-ioniques;
   **(ii)**_{**2**} - les gommes de biopolysaccharides d'origine microbienne telles que
   les gommes de Scléroglucane ou de Xanthane;
   **(ii)**₃ - les gommes issues d'exudats végétaux telles que les gommes Arabique, Tragacanthe, Carrageenane, Agar et Caroube;
   **(ii)**_{**4**}- les alginates;
   **(ii)**_{**5**}- les amidons;
   **(ii)**_{**6**}- les carboxyalkylcelluloses.

Les gommes de guar non ioniques peuvent être modifiées ou non modifiées. Les gommes de guar non modifiées sont par exemple les produits vendus sous la dénomination VIDOGUM GH 175 par la société UNIPECTINE et sous la dénomination JAGUAR C par la société MEYHALL.

Selon la présente invention, on préfère utiliser les gommes de guar non-ioniques modifiées par des groupements hydroxyalkyle en C₁-C₆.
Parmi les groupements hydroxyalkyle, on peut mentionner à titre d'exemple, les groupements hydroxyméthyle, hydroxyéthyle, hydroxypropyle et hydroxybutyle.
Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.
Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

Les gommes de biopolysaccharides d'origine microbienne telles que les gommes de scléroglucane ou de xanthane, les gommes issues d'exudats végétaux telles que les gommes Aabique, gomme Ghatti, gomme Karaya, Tragacanthe, Carrageenane, Agar et Caroube, les hydroxyalkylcelluloses et les carboxyméthylcelluloses, les pectines, les alginates et les amidons sont bien connus de l'homme de l'art et décrits notamment dans l'ouvrage de Robert L. DAVIDSON intitulé "Handbook of Water soluble gums and resins" édité chez Mc Graw Hill Book Company (1980).

Parmi ces gommes, les scléroglucanes plus particulièrement utilisés selon la présente invention, sont représentés par les produits vendus sous la dénomination ACTIGUM CS par la société SANOFI BIO INDUSTRIES et en particulier ACTIGUM CS 11 et sous la dénomination AMIGEL par la société ALBAN MULLER INTERNATIONAL. D'autres scléroglucanes tels que celui traité au glyoxal dans la demande de brevet français N°2633940 peuvent également être utilisés.
Les gommes de Xanthane plus particulièrement utilisés selon la présente invention, sont représentés par les produits vendus sous les dénominations KELTROL, KELTROL T, KELTROL TF, KELTROL BT, KELTROL RD, KELTROL CG par la société NUTRASWEET KELCO, ou sous les dénominations RHODICARE S, RHODICARE H par la société RHODIA CHIMIE.

Parmi les carboxyalkylcelluloses, on utilise de préférence la carboxyméthylcellulose dont on peut citer les produits vendus sous les dénominations BLANOSE 7M8/SF, BLANOSE RAFFINEE 7M, BLANOSE 7LF, BLANOSE 7MF, BLANOSE 9M31F, BLANOSE 12M31XP, BLANOSE 12M31P, BLANOSE 9M31XF, BLANOSE 7H, BLANOSE 7M31, BLANOSE 7H3SXF, par la société AQUALON, ou encore AQUASORB A500 et AMBERGUM 1221, par le société HERCULES, ou encore CELLOGEN HP810A et CELLOGEN HP6HS9, par la société MONTELLO, ou encore PRIMELLOSE par la société AVEBE.

Les polymères épaississants (ii) utilisés dans les compositions de la présente invention sont présents de préférence à raison de 0,01 à 10% environ en poids, en particulier à raison de 0,1 à 5% environ en poids par rapport au poids total de la composition de teinture appliquée sur les fibres kératiniques.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 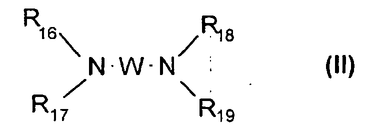 dans laquelle W est un reste propylène éventuellement substitué par un groupement hydroxyle ou un radical alkyle en C₁-C₆; R₁₆ R₁₇ R₁₈ R₁₉ identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques (i) définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques (i) une ou plusieurs bases d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques. Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique (i) et du polymère épaississant (ii) ainsi que des bases d'oxydation, un ou plusieurs coupleurs de façon à modifier ou à enrichir en reflets les nuances obtenues en mettant en oeuvre le ou les colorants direct(s) cationique(s) (i) et la ou les bases d'oxydation.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents tensioactifs, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant le polymère épaississant particulier.

Lorsque l'association du colorant direct cationique (i) et du polymère épaississant (ii) selon l'invention est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante, alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydoréductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique (i) tel que défini précédemment et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le polymère épaississant (ii) tel que défini précédemment.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique (i) tel que défini précédemment et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le polymère épaississant tel que défini précédemment.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) ou (B2) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 à 2:

On a préparé les deux compositions de teinture directe réunies dans le tableau suivant :
*(toutes teneurs exprimées en grammes)*

| | **Exemple 1** | **Exemple 2** |
|---|---|---|
| Colorant direct cationique de formule (I)10 | 0,12 | |
| Colorant direct cationique de formule (I)27 | | 0,1 |
| Gomme de Guar vendue sous la dénomination VIDOGUM GH 175 par la société UNIPECTINE | 1,0 MA* | |
| Gomme de Scléroglucane vendue sous la dénomination AMIGEL par la société ALBAN MULLER INTERNATIONAL | | 1,0 MA* |
| Ethanol | 10 | 10 |
| 2-amino-2-méthyl-1-propanol ....qs | pH 9 | pH 9 |
| Eau déminéralisée ..... qsp | 100 | 100 |

### MA* désigne Matière Active

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| **Exemples** | **Nuances obtenues** |
|---|---|
| 1 | Rouge puissant |
| 2 | Pourpre puissant |

## Revendications

1. Composition pour la teinture des fibres kératiniques et en particulier des fibres kératiniques humaines telles que les cheveux, renfermant dans un milieu approprié pour la teinture, (i)au moins un composé de formule (I) suivant:
A-N=N-B (I)
dans laquelle :
le **symbole A** représente un groupement choisi parmi les structures A₁ à A₃ suivantes : 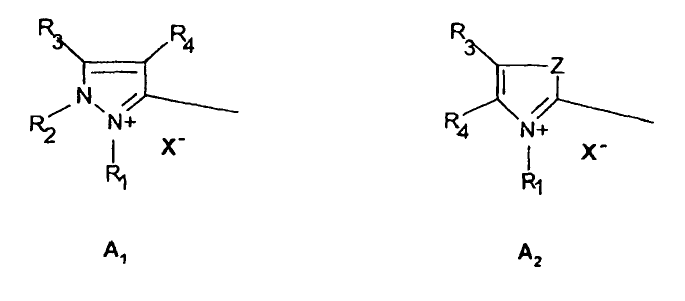 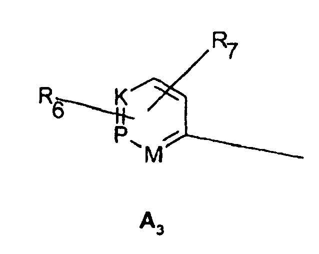 structures A₁ à A₃ dans lesquelles,
R₁ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₂ désigne un radical alkyle en C₁-C₄ ou un radical phényle;
R₃ et R₄, identiques ou différents, représentent un radical alkyle en C₁-C₄ , un radical phényle, ou bien, dans le cas de la structure A1, peuvent former ensemble un cycle benzénique substitué par un ou plusieurs radicaux alkyle en C₁-C₄, alcoxy en C₁-C₄, ou NO₂, et dans le cas de la structure A2, peuvent former ensemble un cycle benzénique éventuellement substitué par un ou plusieurs radicaux alkyle en C₁-C₄, alcoxy en C₁-C₄, ou NO₂;
R₃ peut en outre désigner un atome d'hydrogène;
Z désigne un atome d'oxygène, de soufre ou un groupement -NR₂;
M représente un groupement -CH, -CR (R désignant alkyle en C₁-C₄), ou -NR₅(X⁻)ᵣ;
K représente un groupement -CH, -CR (R désignant alkyle en C₁-C₄), ou -NR₅(X⁻)ᵣ;
P représente un groupement -CH, -CR (R désignant alkyle en C₁-C₄), ou -NR₅(X⁻)ᵣ, r désigne zéro ou 1;
R₅ représente un atome O⁻, un radical alcoxy en C₁-C₄, ou un radical alkyle en C₁-C₄;
R₆ et R₇, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
X- représente un anion de préférence choisi parmi le chlorure, l'iodure, le méthyl sulfate, l'éthyl sulfate, facétate et le perchlorate;
sous réserve que,
si R₄ désigne un radical alkyle en C₁-C₄ et Z désigne un atome de soufre, R₃ ne désigne pas un atome d'hydrogène;
si R₅ désigne O⁻, alors r désigne zéro;
si K ou P ou M désignent -N-alkyle C₁-C₄ X⁻, alors R₆ ou R₇ est différent d'un atome d'hydrogène;
si K désigne -NR₅(X⁻)ₙ alors M= P= -CH; -CR;
si M désigne -NR₅(X⁻)ₙ alors K= P= -CH; -CR;
si P désigne -NR₅(X⁻)ₙ alors K= M et désignent -CH ou -CR;
si Z désigne -NR₂ et R₂ désigne un radical alkyle en C₁-C₄ , alors au moins un des radicaux R₁, R₃ ou
R₄ du groupement de structure **A**_{**2**} est différent d'un radical alkyle en C₁-C₄;
le **symbole B** représente :
- **(a)** un groupement de structure B₁ suivante : 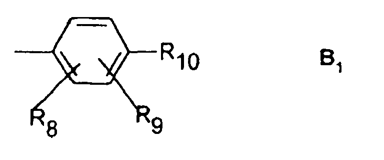 structure B₁ dans laquelle,
R₈ représente un atome d'hydrogène, un atome d'halogène choisi parmi le chlore, le brome, l'iode et le fluor, un radical alkyle en C₁-C₄, alcoxy en C₁-C₄, un radical - OH, -NO₂, -NHR₁₁, -NR₁₂R₁₃, -NHCOalkyle en C₁-C₄, ou forme avec R₉ un cycle à 5 ou 6 chaînons contenant ou non un ou plusieurs hétéroatomes choisis parmi l'azote, l'oxygène ou le soufre;
R₉ représente un atome d'hydrogène, un atome d'halogène choisi parmi le chlore, le brome, l'iode et le fluor, un radical alkyle en C₁-C₄, alcoxy en C₁-C₄, ou forme avec R₁₀ ou R₁₁ un cycle à 5 ou 6 chaînons contenant ou non un ou plusieurs hétéroatomes choisis parmi l'azote, l'oxygène ou le soufre;
R₁₀ représente un atome d'hydrogène, un radical -OH, un radical -NHR₁₁, un radical -NR₁₂R₁₃;
R₁₁ représente un atome d'hydrogène, un radical alkyle en C₁-C₄, un radical monohydroxyalkyle en C₁-C₄, polyhydroxyalkyle en C₂-C₄, un radical phényle; R₁₂ et R₁₃, identiques ou différents, représentent un radical alkyle en C₁-C₄ , un radical monohydroxyalkyle en C₁-C₄, polyhydroxyalkyle en C₂-C₄;
- **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure B2 suivante : 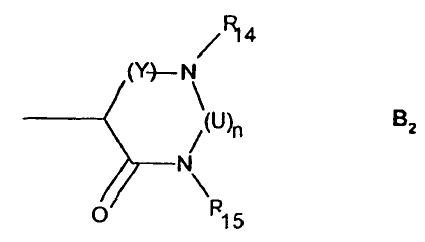 structure B₂ dans laquelle.
R₁₄ et R_{15'} identiques ou différents, représentent un atome d'hydrogène , un radical alkyle en C₁-C₄, un radical phényle;
Y désigne le radical -CO- ou le radical 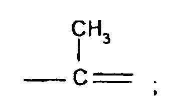 n = 0 ou 1, avec, lorsque n désigne 1, U désigne le radical -CO-
ladite composition étant **caractérisée par le fait qu'**elle contient en outre
**(ii)** au moins un polymère épaississant choisi dans le groupe comprenant :
**(ii)**_{**1**}**-** les gommes de guar non-ioniques;
**(ii)**_{**2**}**-** les gommes de biopolysaccharides d'origine microbienne telles que les gommes de Scléroglucane ou de Xanthane;
**(ii)**_{**3**}**-** les gommes issues d'exudats végétaux telles que les gommes Arabique, Tragacanthe, Carrageenane, Agar et Caroube;
**(ii)**_{**4**}**-** les alginates;
**(II)**_{**5**}**-** les amidons;
**(II)**_{**6**}**-** les carboxyalkylcelluloses.

2. Composition selon la revendication 1, **caractérisée par le fait que** dans la formule (I), les radicaux alkyles en C₁₋C₄ et les radicaux alcoxy en C₁-C₄ sont des radicaux méthyle, éthyle, butyle, méthoxy et éthoxy.

3. Composition selon la revendication 2, **caractérisée par le fait que** les colorants directs cationiques répondent aux structures (I)₁ à (I)₇₇ suivantes :

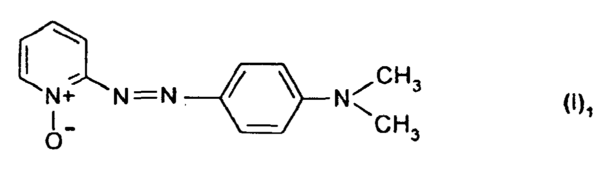

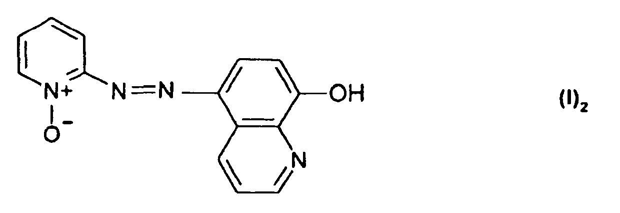

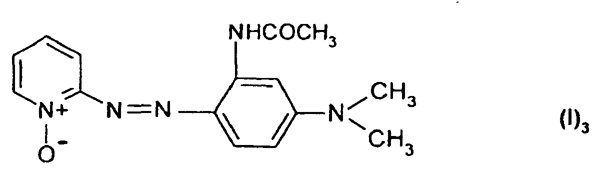

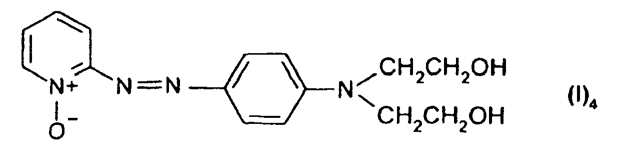

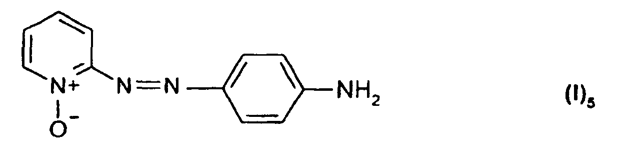

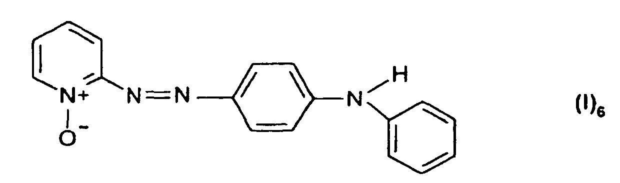

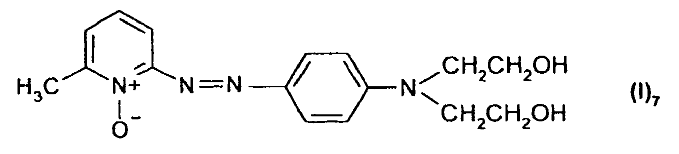

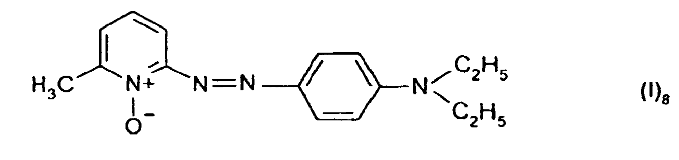

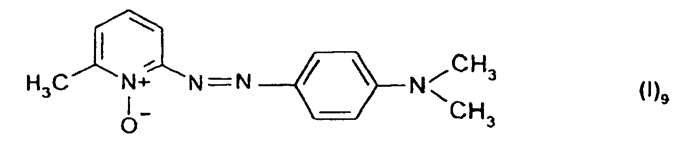

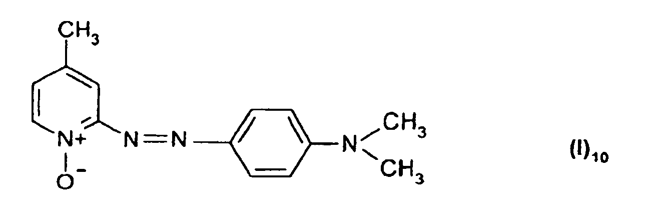

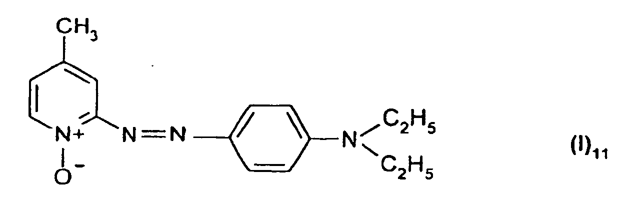

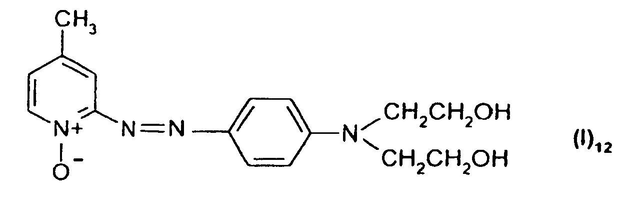

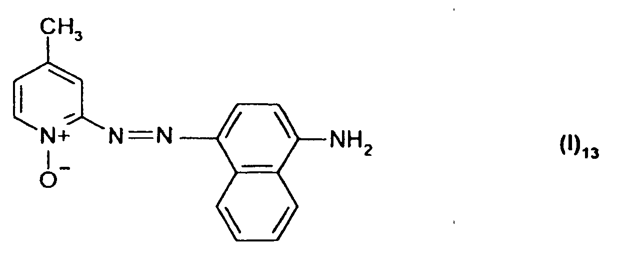

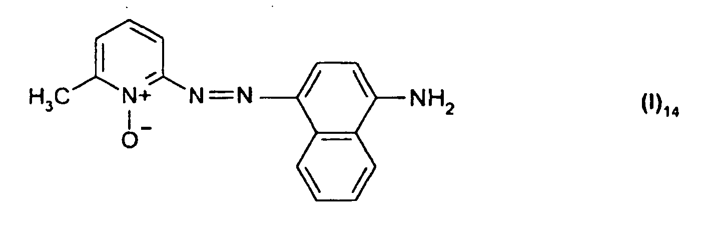

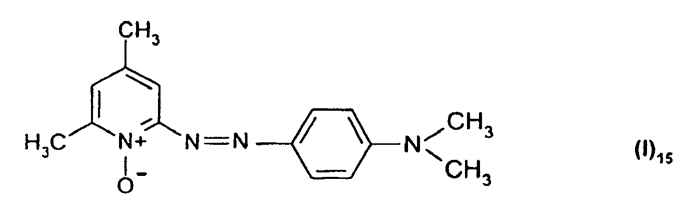

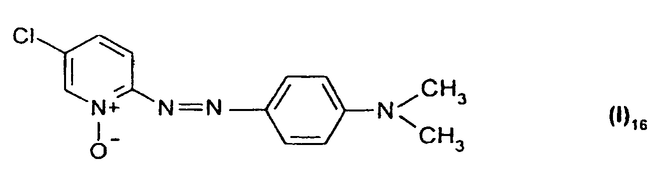

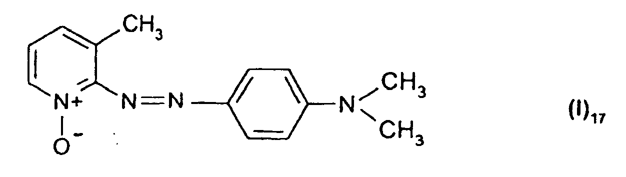

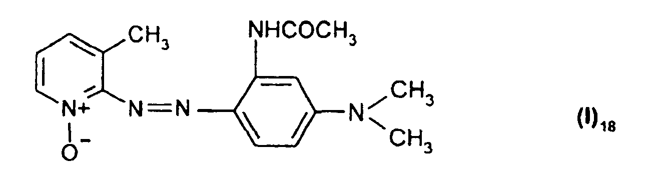

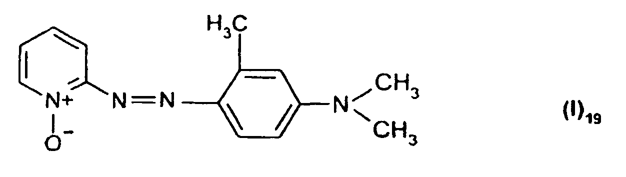

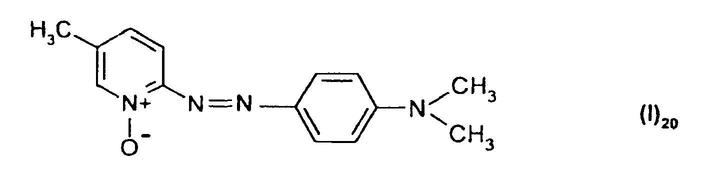

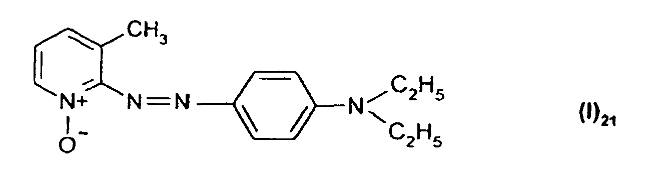

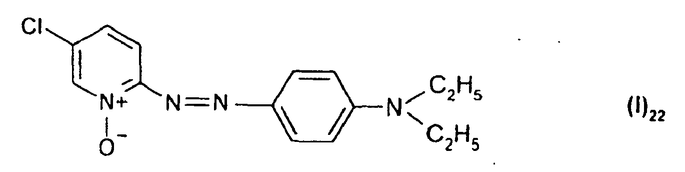

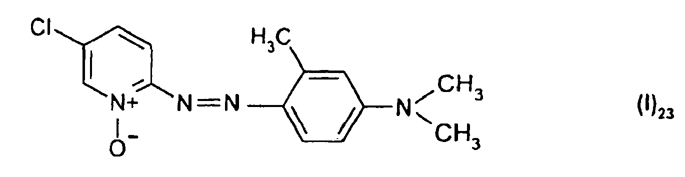

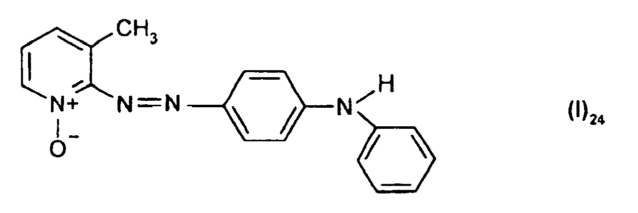

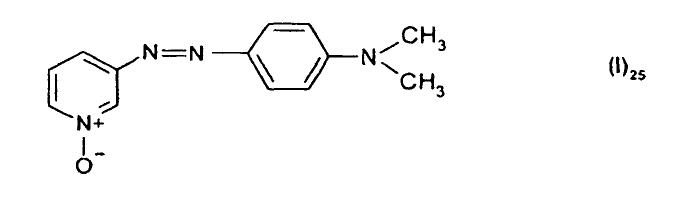

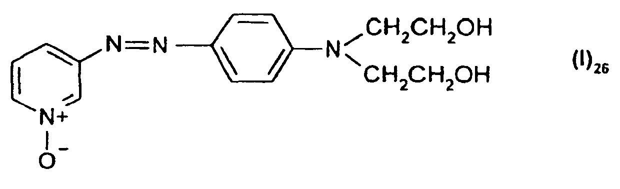

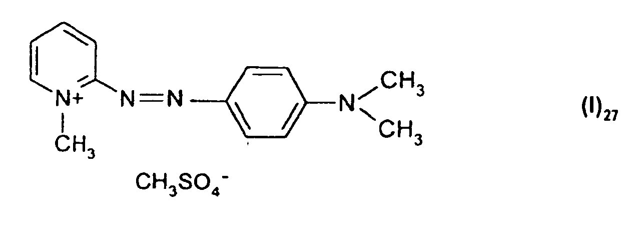

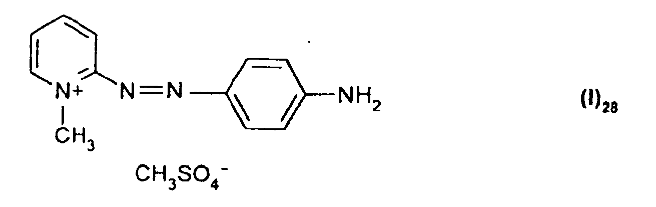

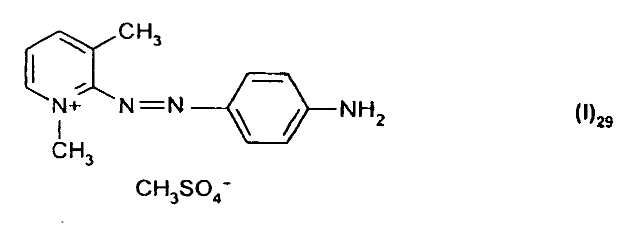

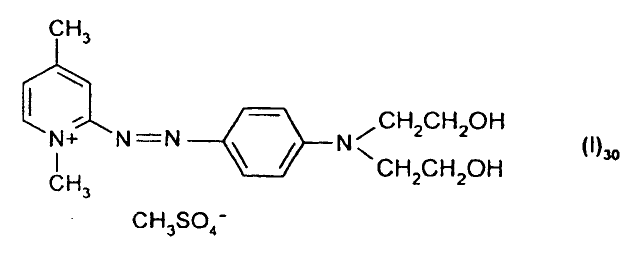

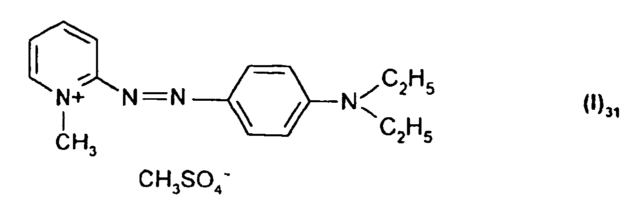

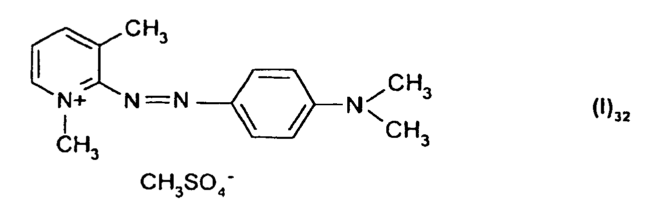

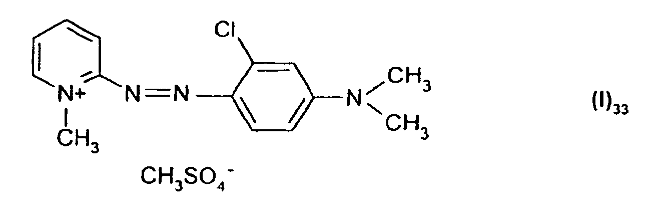

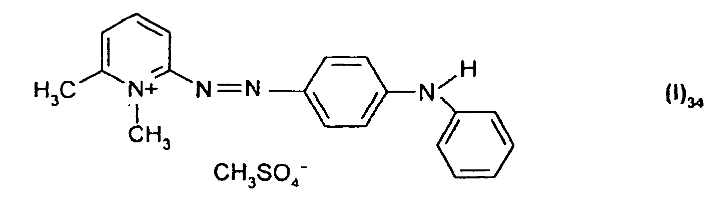

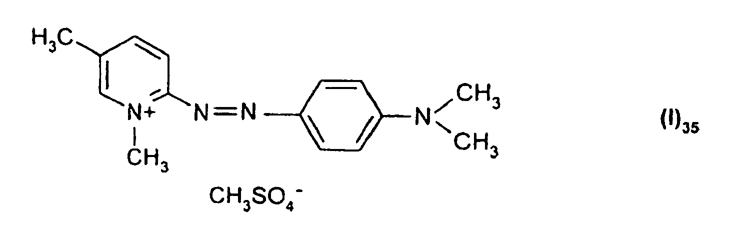

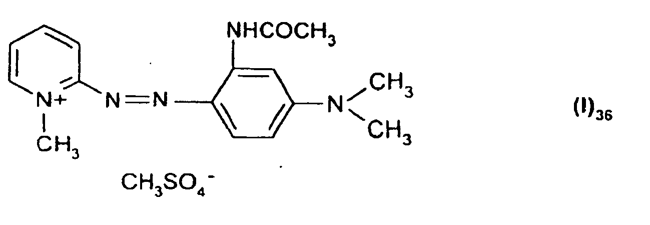

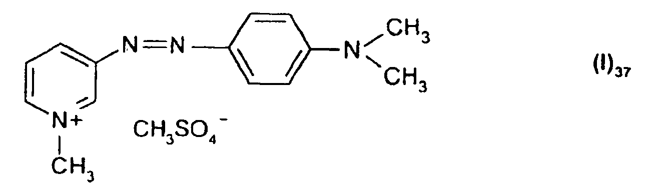

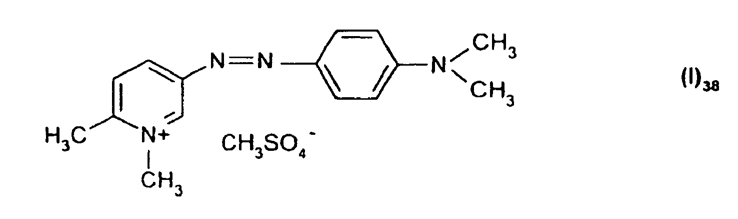

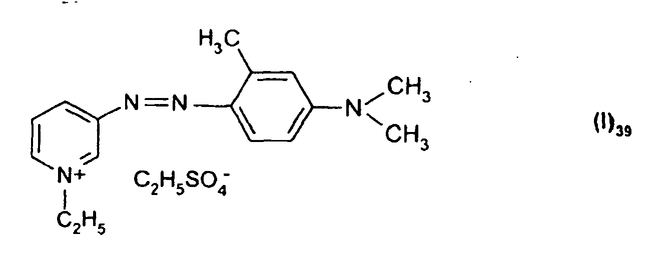

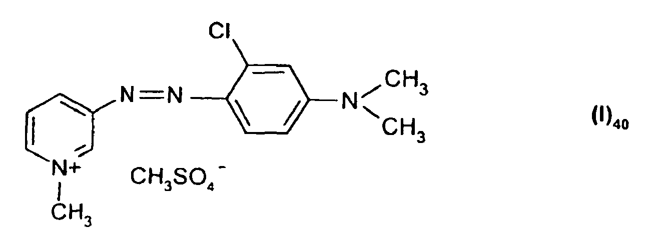

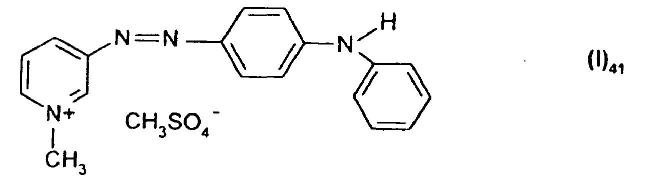

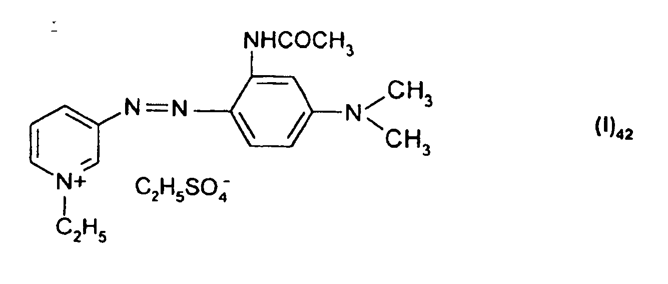

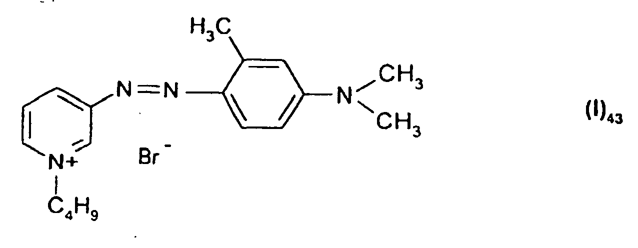

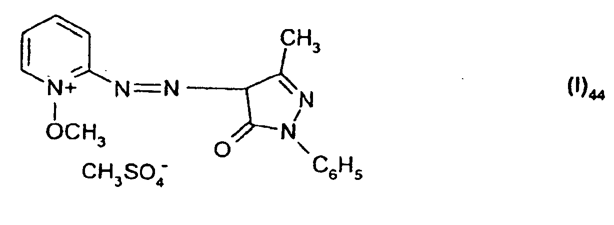

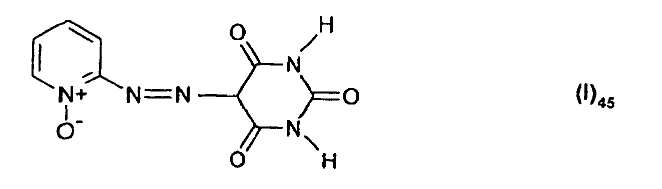

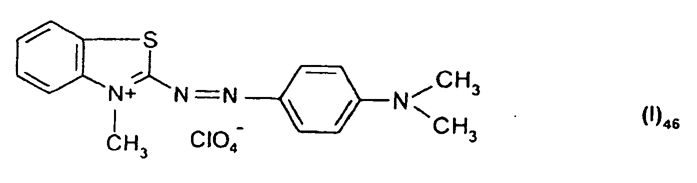

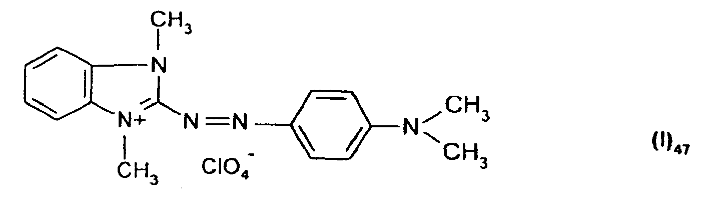

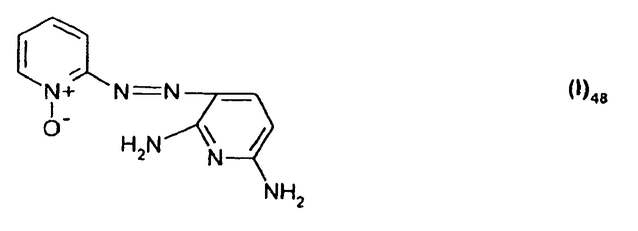

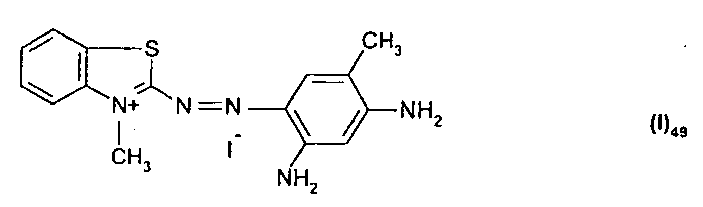

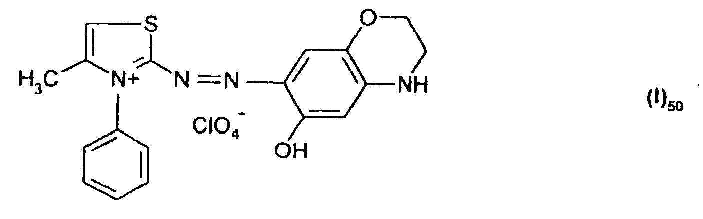

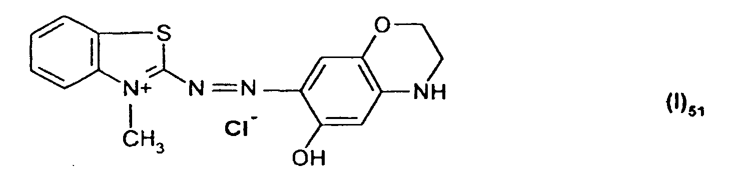

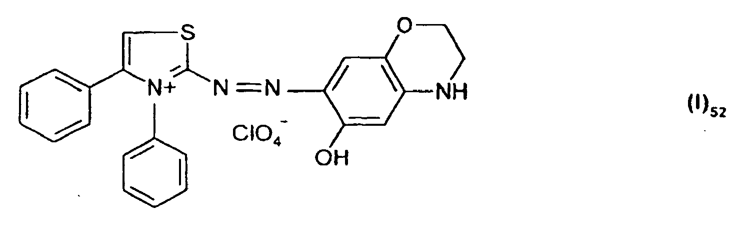

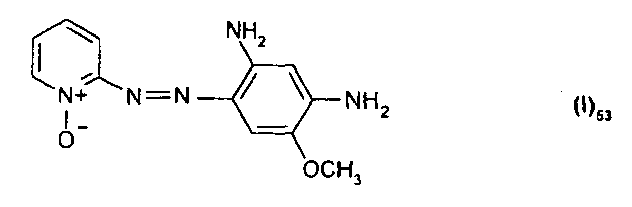

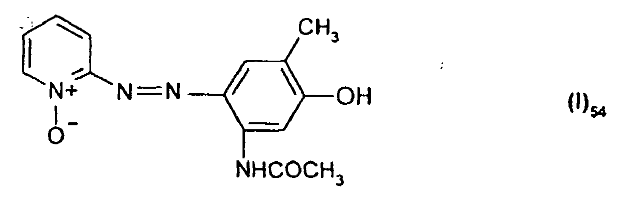

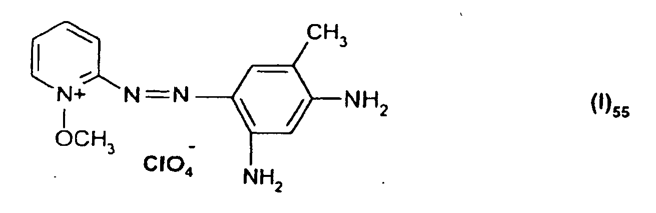

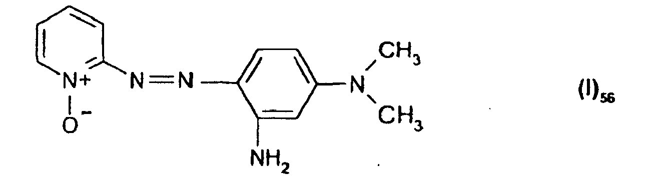

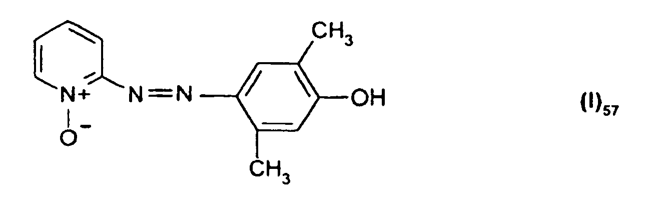

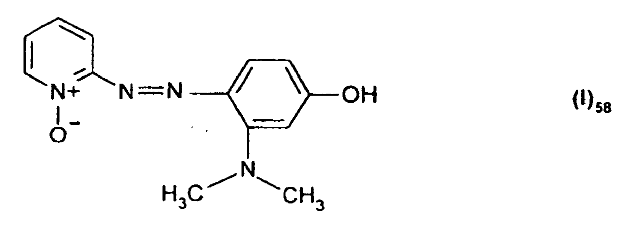

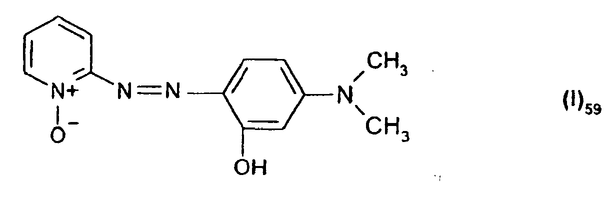

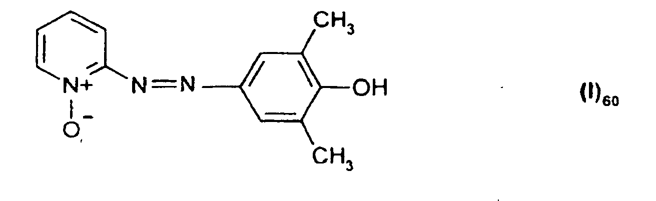

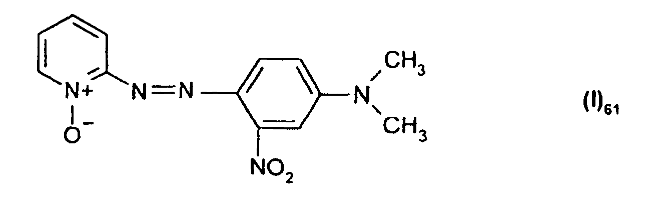

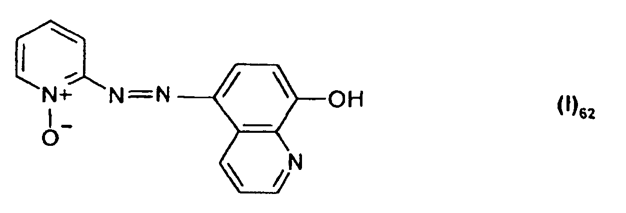

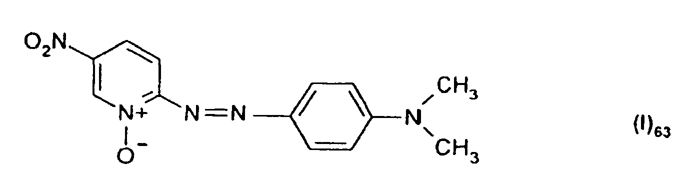

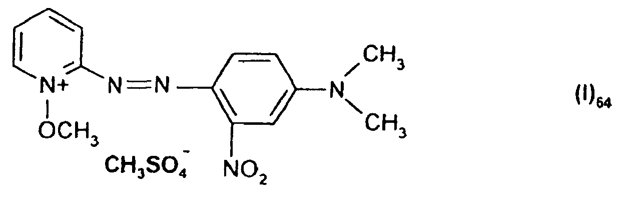

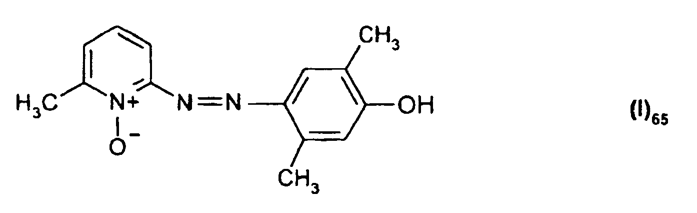

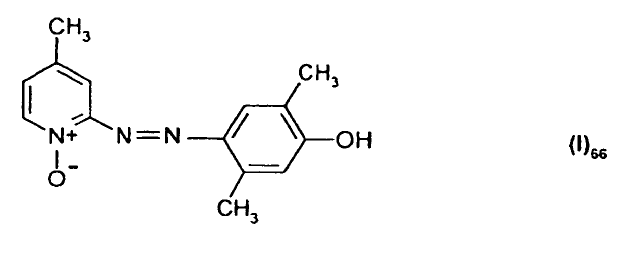

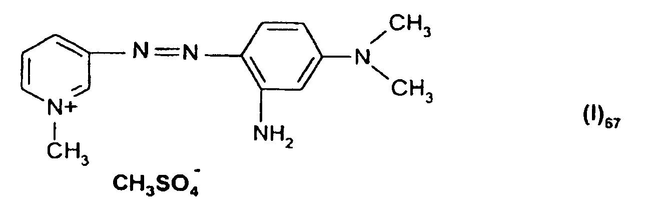

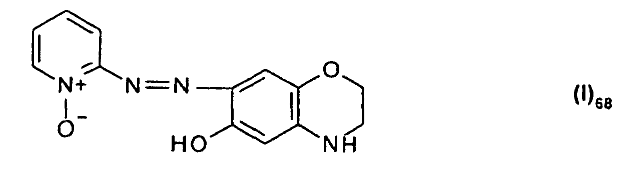

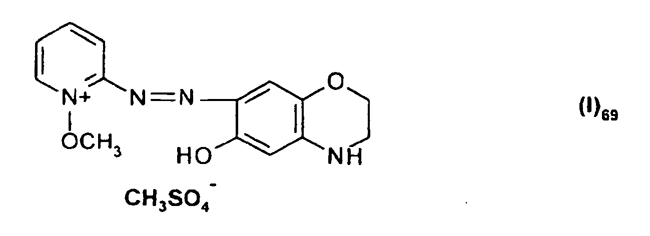

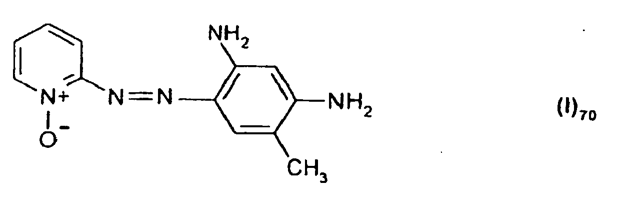

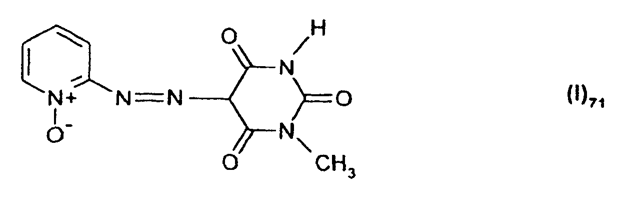

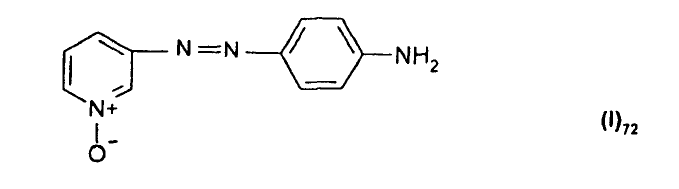

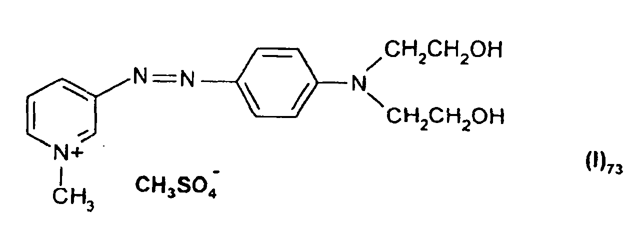

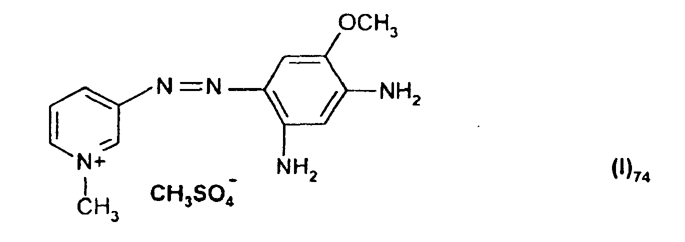

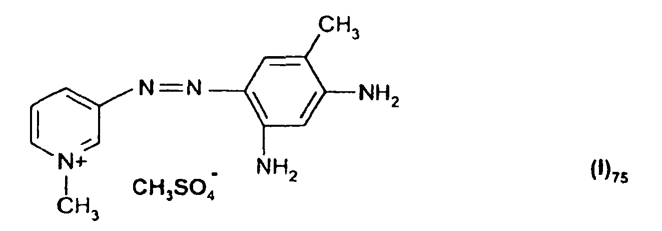

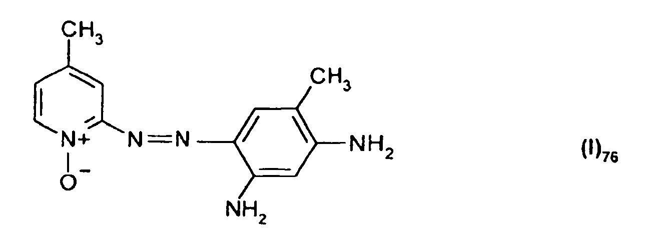

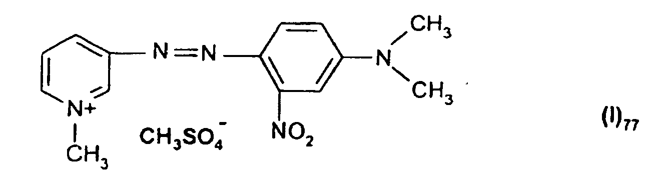

4. Composition selon l'une quelconque des revendications précédentes, **caractérisée par le fait que** le ou les colorants directs cationiques de formule (l), représentent de 0,001 à 10 % en poids du poids total de la composition.

5. Composition selon la revendication 4, **caractérisée par le fait que** le ou les colorants directs cationiques de formule (I), représentent de 0,005 à 5 % en poids du poids total de la composition.

6. Composition selon l'une quelconque des revendications précédentes, **caractérisée par le fait que** le polymère épaississant est une gomme de guar non-ionique modifiée par des groupements hydroxyalkyle en C₁-C₆.

7. Composition selon la revendication 6, **caractérisée par le fait que** la gomme non-ionique présente un taux d'hydroxyalkylation variant entre 0,4 et 1,2.

8. Composition selon l'une quelconque des revendications 1 à 5, **caractérisée par le fait que** le polymère épaississant est une carboxyméthylcellulose.

9. Composition selon l'une quelconque des revendications précédentes, **caractérisée par le fait que** le polymère épaississant (ii) représente de 0,01 à 10 % en poids du poids total de la composition.

10. Composition selon la revendication 9, **caractérisée par le fait que** le polymère épaississant (ii) représente de 0,1 à 5% en poids du poids total de la composition.

11. Composition selon l'une quelconque des revendications précédentes, **caractérisée par le fait que** le milieu approprié pour la teinture (ou support) est constitué par de l'eau ou par un mélange d'eau et d'au moins un solvant organique.

12. Composition selon l'une quelconque des revendications précédentes, **caractérisée par le fait qu'**elle présente un pH compris entre 2 et 11, et de préférence entre 5 et 10.

13. Composition selon l'une quelconque des revendications précédentes, **caractérisée par le fait qu'**elle est destinée à la teinture d'oxydation et qu'elle contient une ou plusieurs bases d'oxydation choisie parmi les paraphénylènediamines, les bis-phénylalkylènediamines, les para-aminophénols, les ortho-aminophénols et les bases hétérocycliques.

14. Composition selon la revendication 13, **caractérisée par le fait que** la ou les bases d'oxydation représentent 0,0005 à 12 % en poids du poids total de la composition tinctoriale.

15. Composition selon la revendication 14, **caractérisée par le fait que** la ou les bases d'oxydation représentent 0,005 à 6 % en poids du poids total de la composition tinctoriale.

16. Composition selon l'une quelconque des revendications 13 à 15, **caractérisée par le fait qu'**elle renferme un ou plusieurs coupleurs choisis parmi les métaphénylènediamines, les méta-aminophénols, les métadiphénols et les coupleurs hétérocycliques.

17. Composition selon la revendication 16, **caractérisée par le fait que** le ou les coupleurs représentent de 0,0001 à 10 % en poids du poids total de la composition tinctoriale.

18. Composition selon la revendication 17, **caractérisée par le fait que** le ou les coupleurs représentent de 0,005 à 5 % en poids du poids total de la composition tinctoriale.

19. Composition selon l'une quelconque des revendications précédentes, **caractérisée par le fait qu'**elle est destinée à la teinture d'oxydation ou la teinture directe éclaircissante et qu'elle renferme alors au moins un agent oxydant.

20. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 19, pendant un temps suffisant pour développer la coloration désirée, après quoi on rince, on lave éventuellement au shampooing, on rince à nouveau et on sèche.

21. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 19, pendant un temps suffisant pour développer la coloration désirée, sans rinçage final.

22. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique (i) tel que défini dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le polymère épaississant (ii) tel que défini dans les revendications précédentes.

23. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique (i) tel que défini dans les revendications précédentes et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le polymère épaississant (ii) tel que défini dans les revendications précédentes.

24. Dispositif à plusieurs compartiments ou "kit" de teinture à plusieurs compartiments, **caractérisé par le fait qu'**un premier compartiment renferme la composition (A1) ou (A2) telle que définie à la revendication 22 ou 23 et un second compartiment renferme la composition (B1) ou (B2) telle que définie à la revendication 22 ou 23.

## Claims

1. Composition for dyeing keratin fibres, and in particular human keratin fibres such as the hair, containing, in a medium which is suitable for dyeing, (i) at least one compound of formula (I) below:
**A-N=N-B** (I)
in which:
**the symbol A** represents a group chosen from the structures A₁ to A₃ below: 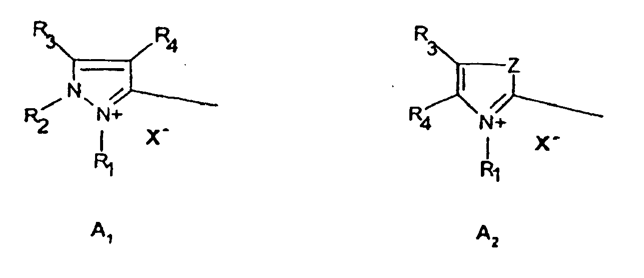 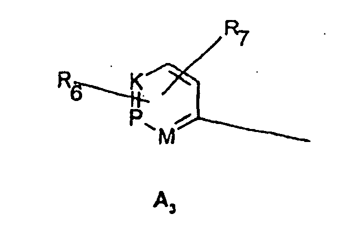 in which structures A₁ to A₃,
R₁ denotes a C₁-C₄ alkyl radical, a phenyl radical which can be substituted with a C₁-C₄ alkyl radical or a halogen atom chosen from chlorine, bromine, iodine and fluorine;
R₂ denotes a C₁-C₄ alkyl radical or a phenyl radical;
R₃ and R₄, which may be identical or different, represent a C₁-C₄ alkyl radical, a phenyl radical or else, in the case of structure A1, can together form a benzene ring substituted with one or more C₁-C₄ alkyl, C₁-C₄ alkoxy or NO₂ radicals and, in the case of structure A2, can together form a benzene ring optionally substituted with one or more C₁-C₄ alkyl, C₁-C₄ alkoxy or NO₂ radicals;
R₃ can also denote a hydrogen atom;
Z denotes an oxygen or sulphur atom or a group -NR₂;
M represents a -CH, -CR (R denoting C₁-C₄ alkyl) or -NR₅ (X⁻)ᵣ group;
K represents a -CH, -CR (R denoting C₁-C₄ alkyl) or -NR₅ (X⁻)ᵣ group;
P represents a -CH, -CR (R denoting C₁-C₄ alkyl) or -NR₅(X⁻)ᵣ group; r denotes zero or 1;
R₅ represents an atom O⁻, a C₁-C₄ alkoxy radical or a C₁-C₄ alkyl radical;
R₆ and R_{7,} which may be identical or different, represent a hydrogen atom, a halogen atom chosen from chlorine, bromine, iodine and fluorine, a C₁₋C₄ alkyl or C₁-C₄ alkoxy radical or an -NO₂ radical; X- represents an anion preferably chosen from chloride, iodide, methyl sulphate, ethyl sulphate, acetate and perchlorate;
with the proviso that,
if R₄ denotes a C₁-C₄ alkyl radical and Z denotes a sulphur atom, R₃ does not denote a hydrogen atom;
if R₅ denotes O⁻, then r denotes zero;
if K or P or M denote C₁-C₄ -N-alkyl X⁻, then R₆ or R₇ is other than a hydrogen atom;
if K denotes -NR₅ (X⁻)ᵣ, then M=P= -CH, -CR;
if M denotes -NR₅(X⁻)ᵣ, then K=P= -CH, -CR;
if P denotes -NR₅(X⁻)ᵣ, then K=M and denote -CH or -CR;
if Z denotes -NR₂ and R₂ denotes a C₁-C₄ alkyl radical, then at least one of the radicals R₁, R₃ or R₄ of the group of structure **A**_{**2**} is other than a C₁-C₄ alkyl radical;
**the symbol B** represents:
- **(a)** a group of structure B₁ below: 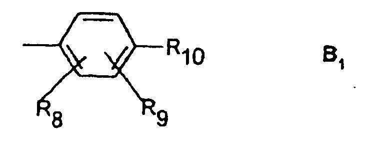 in which structure B₁,
R₈ represents a hydrogen atom, a halogen atom chosen from chlorine, bromine, iodine and fluorine, a C₁-C₄ alkyl or C₁-C₄ alkoxy radical, a radical -OH, -NO₂, -NHR₁₁, -NR₁₂R₁₃, -NHCO (C₁-C₄) alkyl, or forms with R₉ a 5- or 6-membered ring which may or may not contain one or more hetero atoms chosen from nitrogen, oxygen and sulphur;
R₉ represents a hydrogen atom, a halogen atom chosen from chlorine, bromine, iodine and fluorine, a C₁-C₄ alkyl or C₁-C₄ alkoxy radical or forms, with R₁₀ or R₁₁, a 5- or 6-membered ring which may or may not contain one or more hetero atoms chosen from nitrogen, oxygen and sulphur;
R₁₀ represents a hydrogen atom, an -OH radical, a radical -NHR₁₁ or a radical -NR₁₂R₁₃;
R₁₁ represents a hydrogen atom, a C₁-C₄ alkyl radical, a C₁-C₄ monohydroxyalkyl radical, a C₂-C₄ polyhydroxyalkyl radical or a phenyl radical;
R₁₂ and R₁₃, which may be identical or different, represent a C₁-C₄ alkyl radical, a C₁-C₄ monohydroxyalkyl radical or a C₂-C₄ polyhydroxyalkyl radical;
- **(b)** a 5- or 6-membered nitrogenous heterocyclic group which can contain other hetero atoms and/or carbonyl groups and which can be substituted with one or more C₁-C₄ alkyl, amino or phenyl radicals, and in particular a group of structure B₂ below: 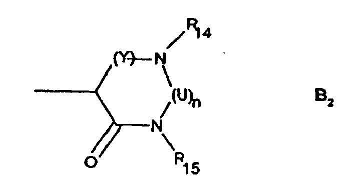
in which structure B₂,
R₁₄ and R₁₅, which may be identical or different, represent a hydrogen atom, a C₁-C₄ alkyl radical or a phenyl radical;
Y denotes the -CO- radical or the radical 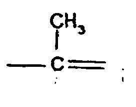 n = 0 or 1, with, when n denotes 1, U denoting a -CO- radical,
the said composition being **characterized in that** it also contains
**(ii)** at least one thickening polymer chosen from the group comprising:
**(ii)**_{**1**} **-** nonionic guar gums;
**(ii)**_{**2**} **-** biopolysaccharide gums of microbial origin, such as scleroglucan gum or xanthan gum;
**(ii)**_{**3**} **-** gums derived from plant exudates, such as gum arabic, gum tragacanth, carrageenan gum, agar gum and carob gum;
**(ii)**_{**4**} **-** alginates;
**(ii)**_{**5**} **-** starches;
**(ii)**_{**6**} **-** carboxyalkylcelluloses.

2. Composition according to Claim 1, **characterized in that** in formula (I), the C₁-C₄ alkyl radicals and the C₁-C₄ alkoxy radicals are methyl, ethyl, butyl, methoxy and ethoxy radicals.

3. Composition according to Claim 2, **characterized in that** the cationic direct dyes correspond to the structures (I)₁ to (I)₇₇ below:

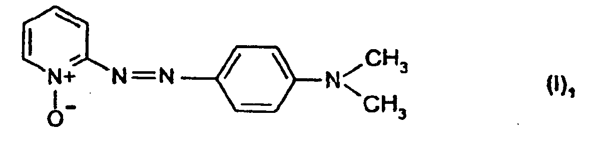

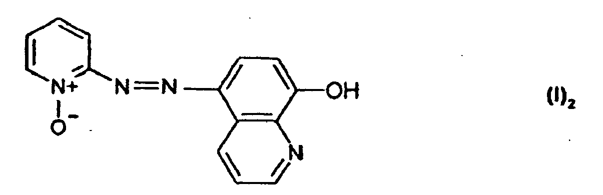

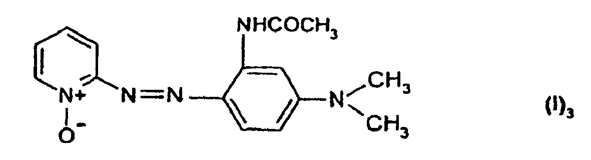

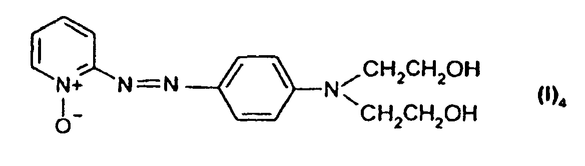

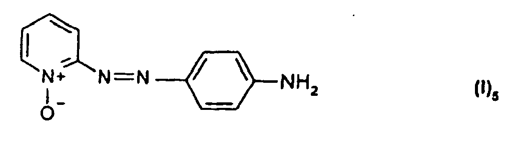

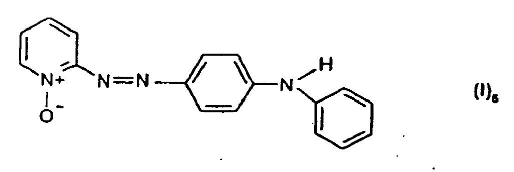

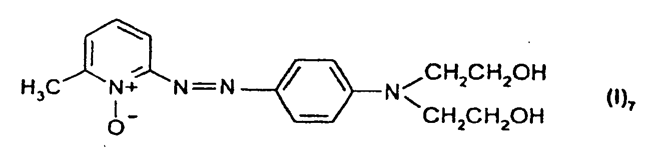

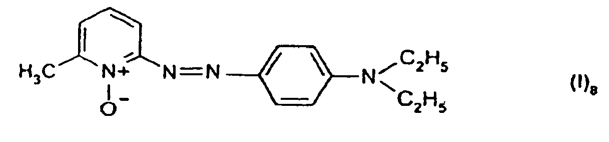

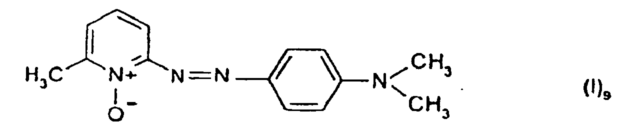

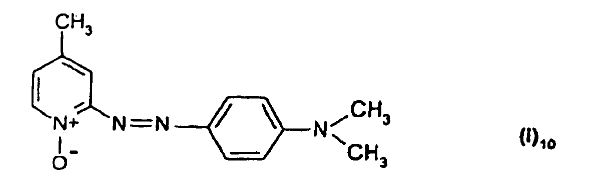

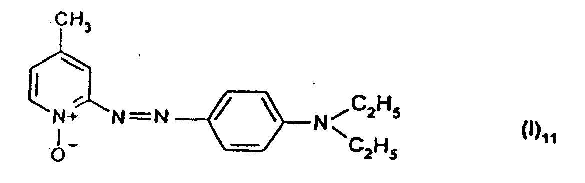

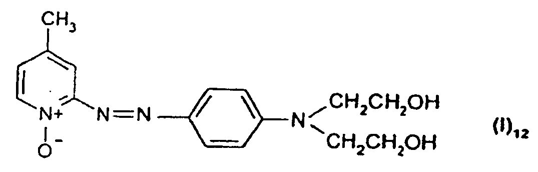

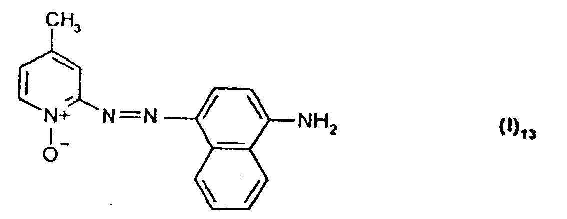

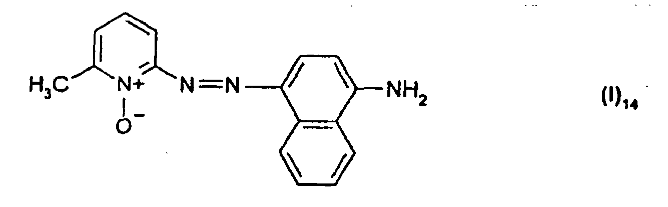

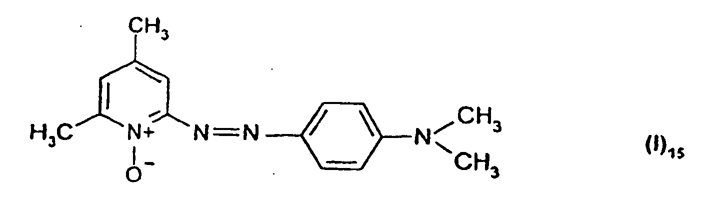

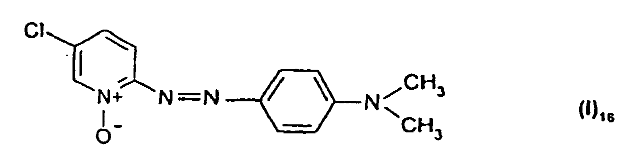

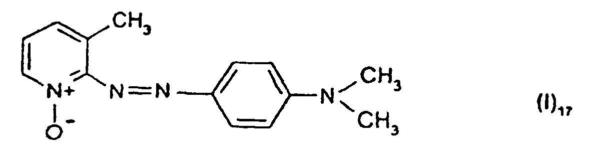

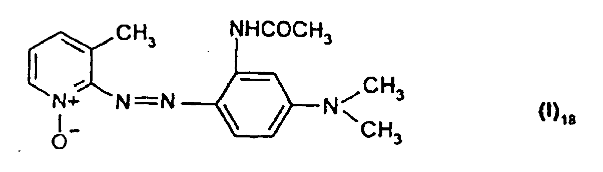

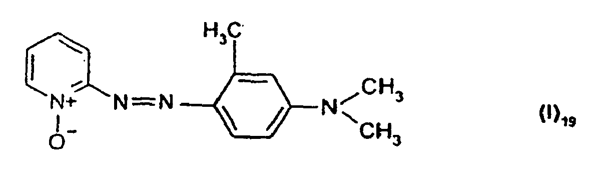

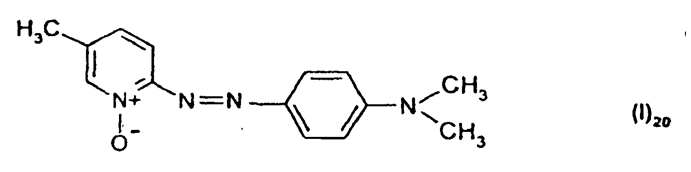

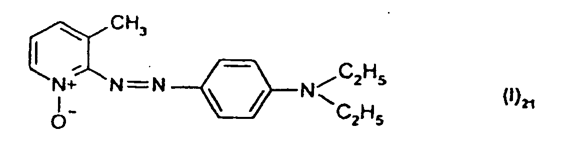

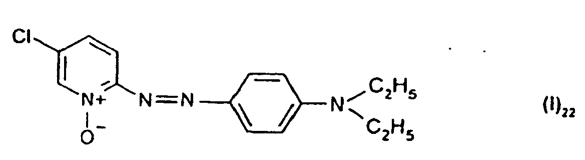

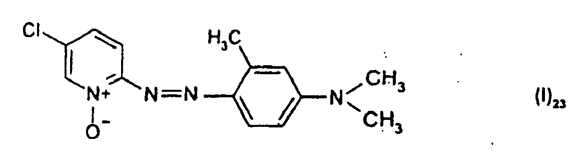

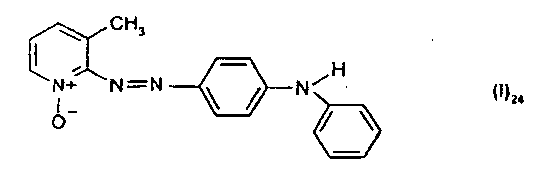

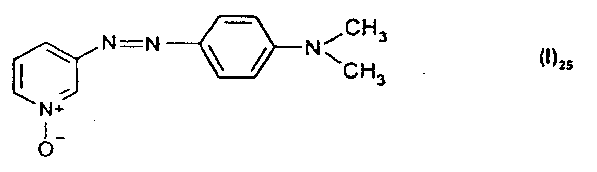

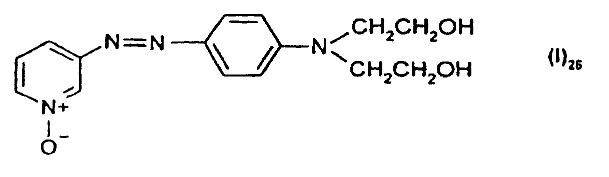

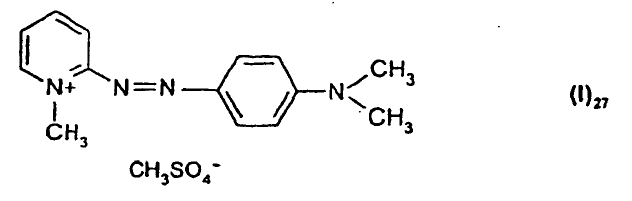

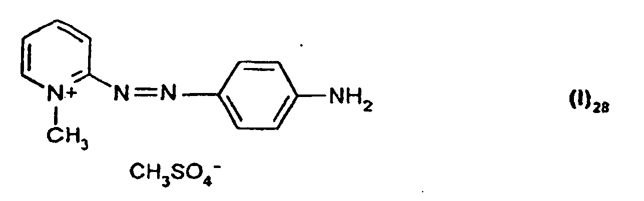

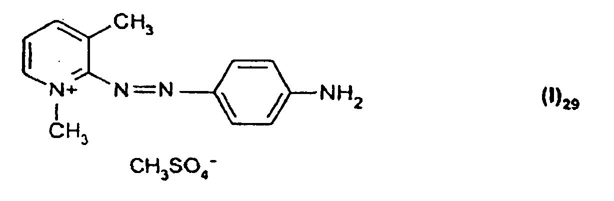

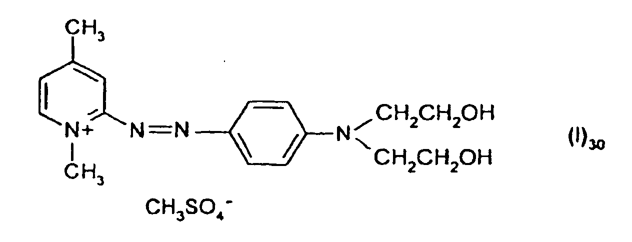

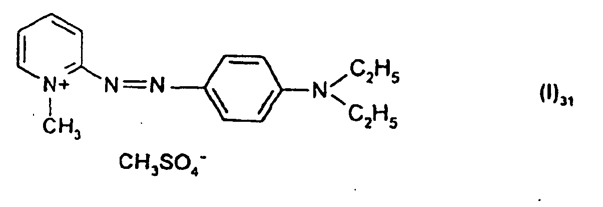

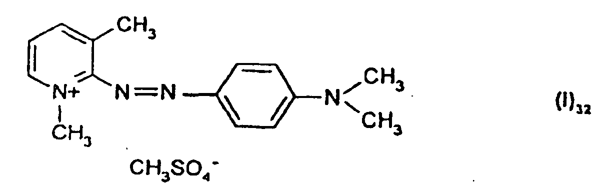

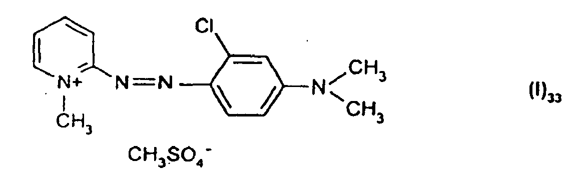

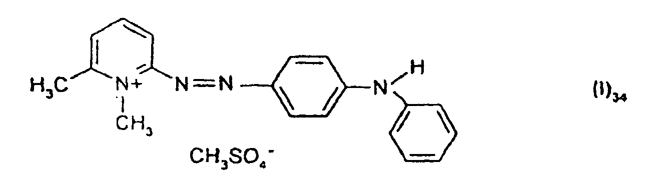

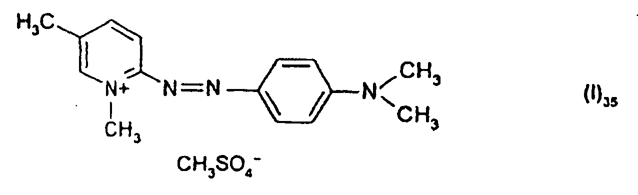

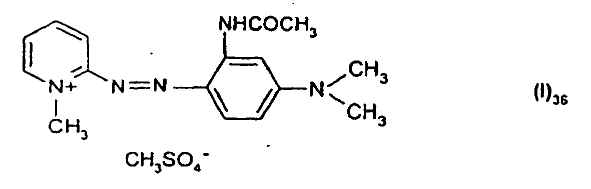

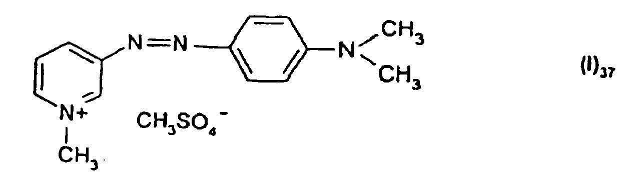

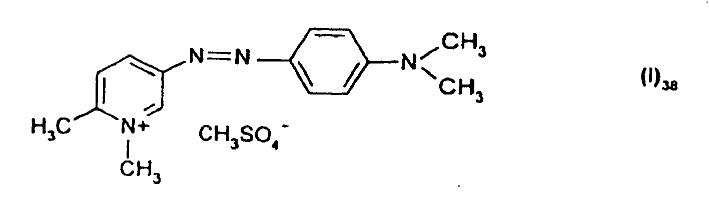

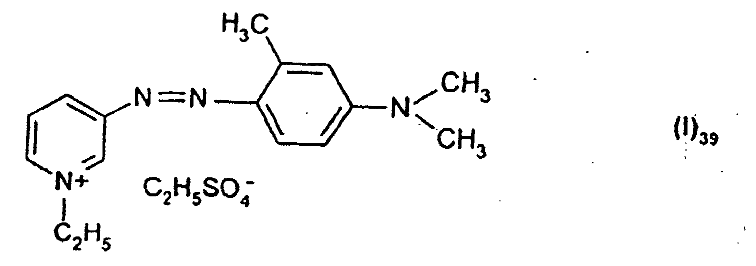

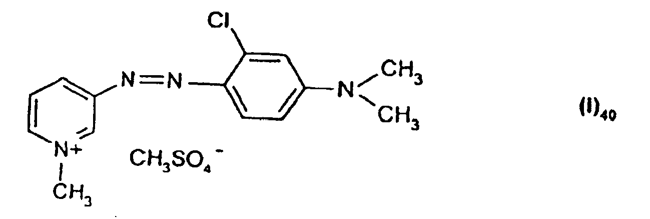

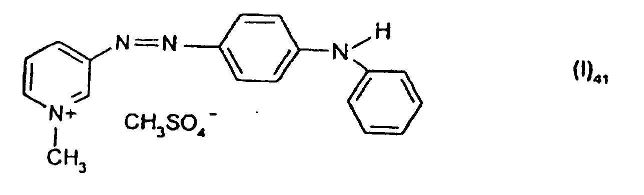

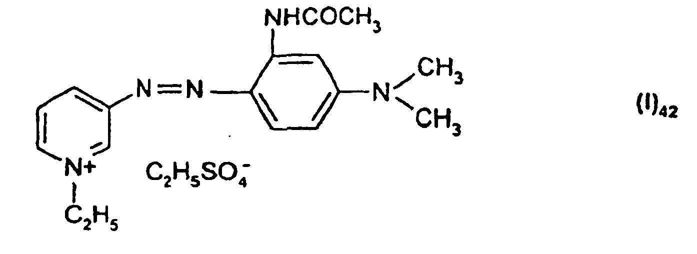

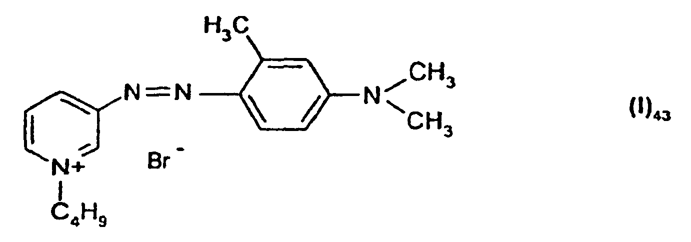

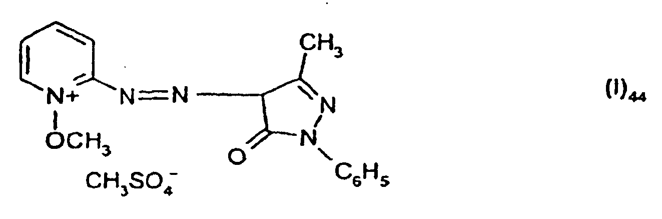

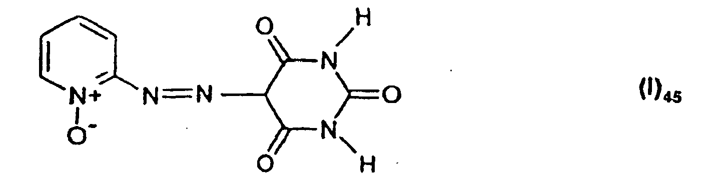

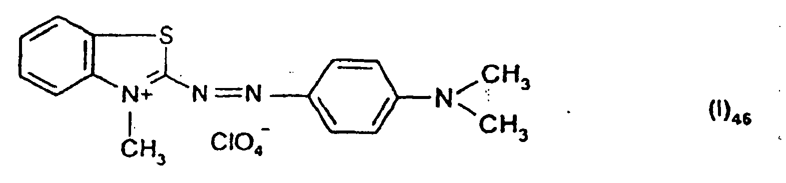

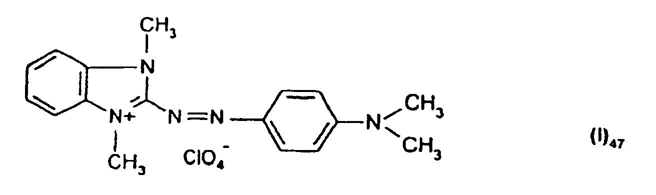

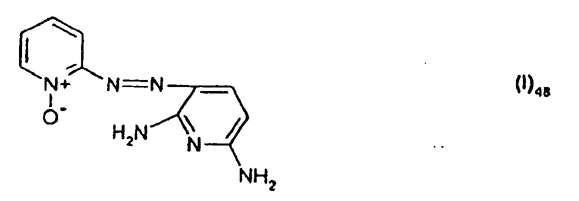

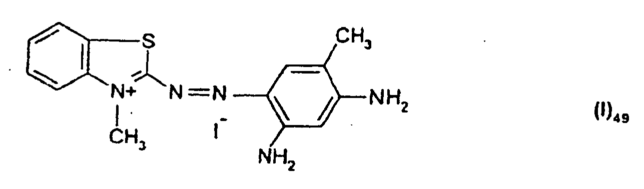

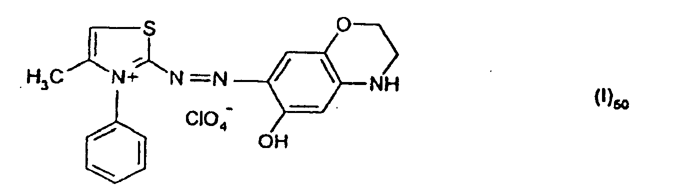

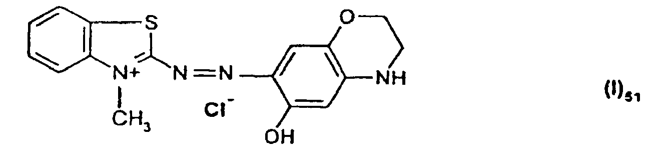

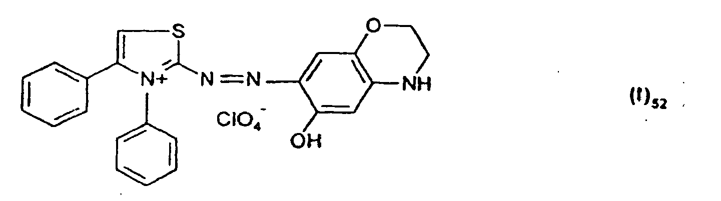

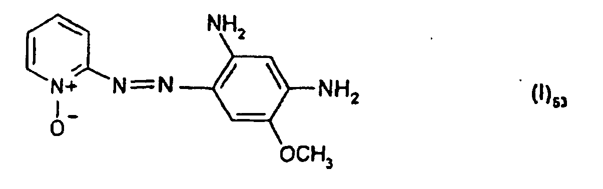

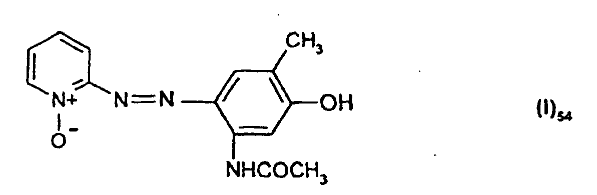

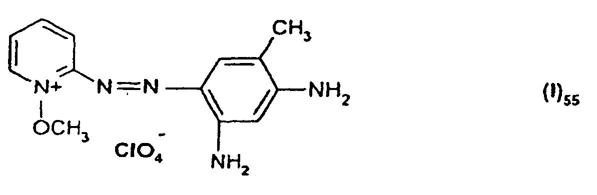

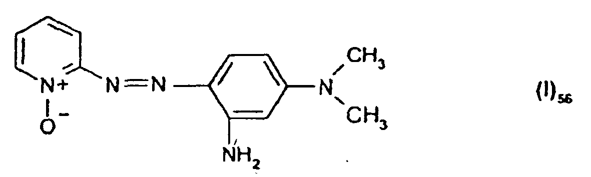

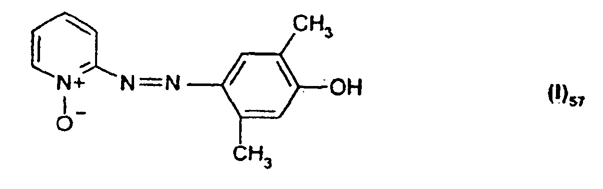

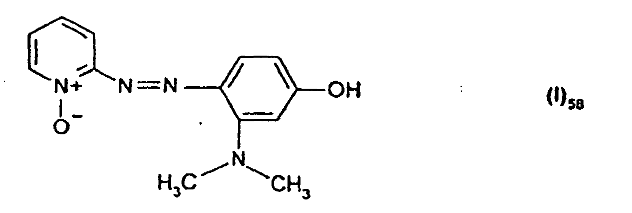

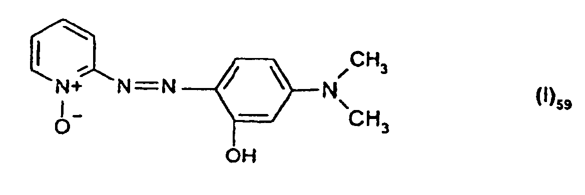

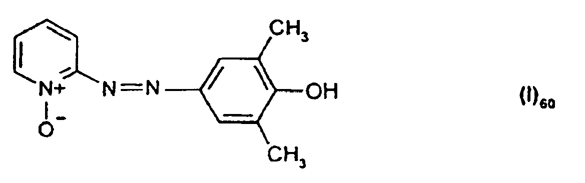

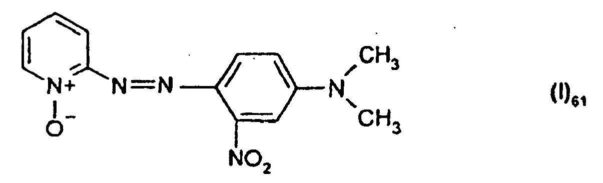

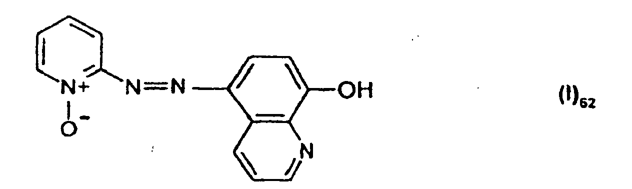

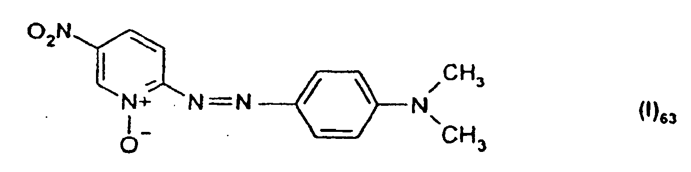

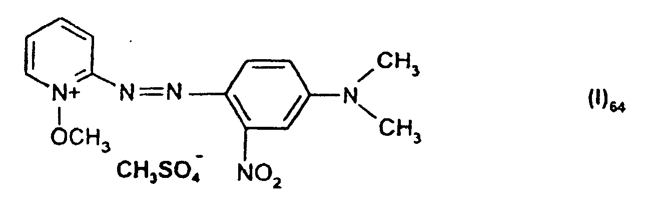

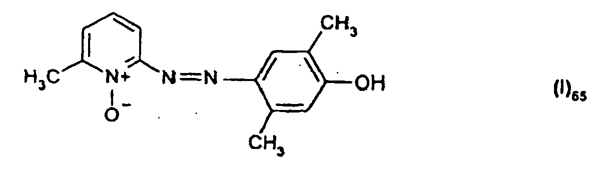

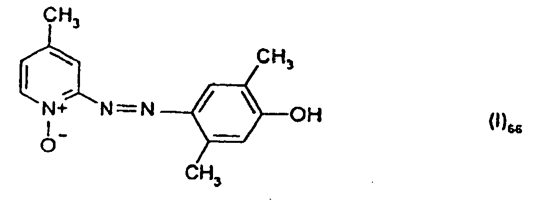

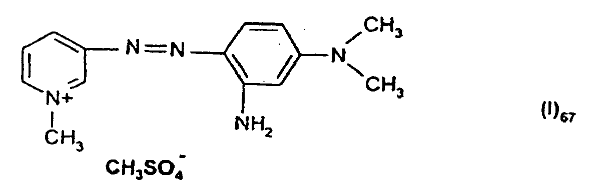

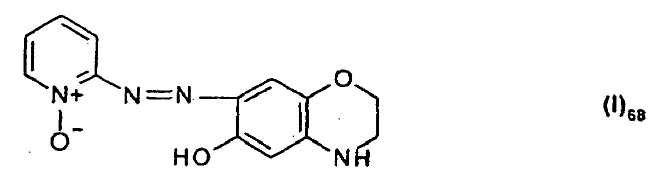

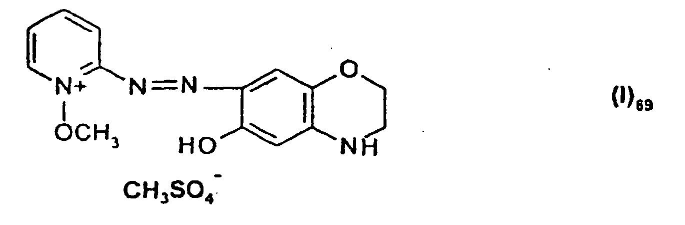

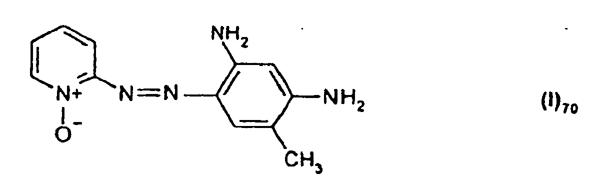

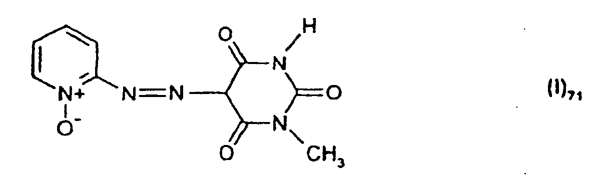

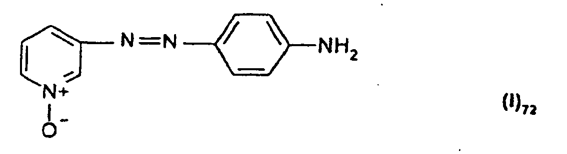

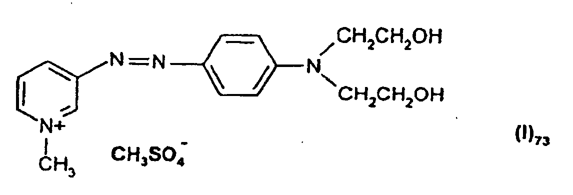

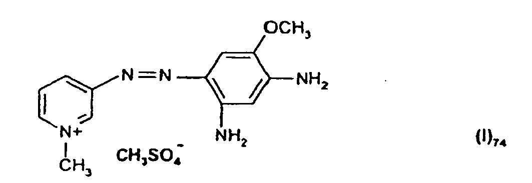

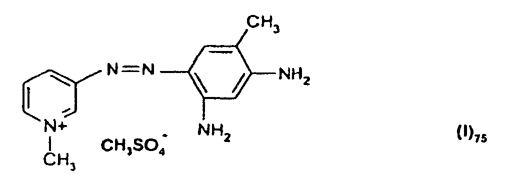

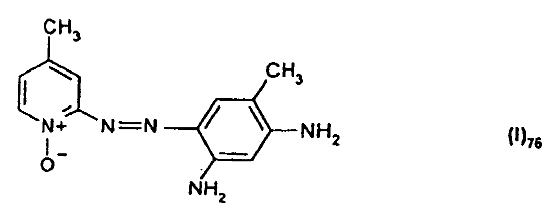

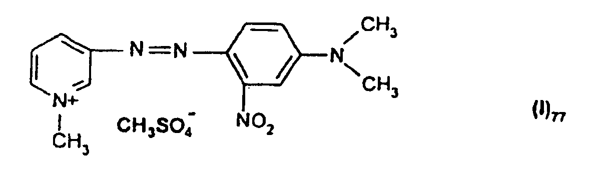

4. Composition according to any one of the preceding claims, **characterized in that** the cationic direct dye(s) of formula (I) represent(s) from 0.001 to 10% by weight relative to the total weight of the composition.

5. Composition according to Claim 4, **characterized in that** the cationic direct dye(s) of formula (I) represent(s) from 0.005 to 5% by weight relative to the total weight of the composition.

6. Composition according to any one of the preceding claims, **characterized in that** the thickening polymer is a nonionic guar gum modified with C₁-C₆ hydroxyalkyl groups.

7. Composition according to Claim 6, **characterized in that** the nonionic gum has a degree of hydroxylation ranging between 0.4 and 1.2.

8. Composition according to any one of Claims 1 to 5; **characterized in that** the thickening polymer is a carboxymethylcellulose.

9. Composition according to any one of the preceding claims, **characterized in that** the thickening polymer (ii) represents from 0.01 to 10% by weight relative to the total weight of the composition..

10. Composition according to Claim 9, **characterized in that** the thickening polymer (ii) represents from 0.1 to 5% by weight relative to the total weight of the composition.

11. Composition according to any one of the preceding claims, **characterized in that** the medium which is suitable for dyeing (or support) consists of water or of a mixture of water and at least one organic solvent.

12. Composition according to any one of the preceding claims, **characterized in that** it has a pH of between 2 and 11 and preferably between 5 and 10.

13. Composition according to any one of the preceding claims, **characterized in that** it is intended for oxidation dyeing and **in that** it contains one or more oxidation bases chosen from para-phenylenediamines, bis(phenyl)alkylenediamines, para-aminophenols, ortho-aminophenols and heterocyclic bases.

14. Composition according to Claim 13, **characterized in that** the oxidation base(s) represent(s) from 0.0005 to 12% by weight relative to the total weight of the dye composition.

15. Composition according to Claim 14, **characterized in that** the oxidation base(s) represent(s) from 0.005 to 6% by weight relative to the total weight of the dye composition.

16. Composition according to any one of Claims 13 to 15, **characterized in that** it contains one or more couplers chosen from meta-phenylenediamines, meta-aminophenols, meta-diphenols and heterocyclic couplers.

17. Composition according to Claim 16, **characterized in that** the coupler(s) represent(s) from 0.0001 to 10% by weight relative to the total weight of the dye composition.

18. Composition according to Claim 17, **characterized in that** the coupler (s) represent(s) from 0.005 to 5% by weight relative to the total weight of the dye composition.

19. Composition according to any one of the preceding claims, **characterized in that** it is intended for oxidation dyeing or lightening direct dyeing and **in that** it contains at least one oxidizing agent.

20. Process for dyeing keratin fibres, and in particular human keratin fibres such as the hair, **characterized in that** at least one dye composition as defined in any one of Claims 1 to 19 is applied to the fibres, for a period which is sufficient to develop the desired coloration, after which the fibres are rinsed, optionally washed with shampoo, rinsed again and dried.

21. Process for dyeing keratin fibres, and in particular human keratin fibres such as the hair, **characterized in that** at least one dye composition as defined in any one of Claims 1 to 19 is applied to the fibres, for a period which is sufficient to develop the desired coloration, without final rinsing.

22. Process for dyeing keratin fibres, and in particular human keratin fibres such as the hair, **characterized in that** it comprises a first step which consists in separately storing, on the one hand, a composition (A1) comprising, in a medium which is suitable for dyeing, at least one cationic direct dye (i) as defined in the preceding claims and at least one oxidation base, and, on the other hand, a composition (B1) comprising, in a medium which is suitable for dyeing, at least one oxidizing agent, and then in mixing them together at the time of use, after which this mixture is applied to the keratin fibres, the composition (A1) or the composition (B1) containing the thickening polymer (ii) as defined in the preceding claims.

23. Process for dyeing keratin fibres, and in particular human keratin fibres such as the hair, **characterized in that** it comprises a first step which consists in separately storing, on the one hand, a composition (A2) comprising, in a medium which is suitable for dyeing, at least one cationic direct dye (i) as defined in the preceding claims and, on the other hand, a composition (B2) comprising, in a medium which is suitable for dyeing, at least one oxidizing agent, and then in mixing them together at the time of use, after which this mixture is applied to the keratin fibres, the composition (A2) or the composition (B2) containing the thickening polymer (ii) as defined in the preceding claims.

24. Multi-compartment dyeing device or multi-compartment dyeing "kit", **characterized in that** a first compartment comprises the composition (A1) or (A2) as defined in Claim 22 or 23 and a second compartment comprises the composition (B1) or (B2) as defined in Claim 22 or 23.

## Patentansprüche

1. Zusammensetzung zum Färben von Keratinfasern und insbesondere menschlichen Keratinfasern, wie Haaren, die in einem zum Färben geeigneten Medium enthält: (i) mindestens eine Verbindung der folgenden Formel (I):
A-N=N-B (I)
worin bedeuten:
das Symbol A eine Gruppe, die unter den folgenden Strukturen A₁ bis A₃ ausgewählt ist: 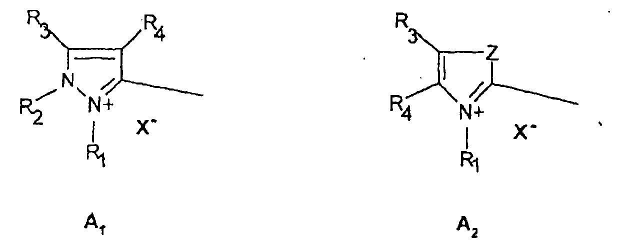 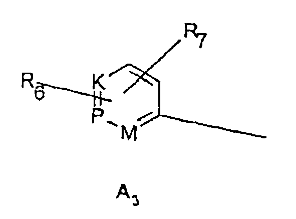
wobei in den Strukturen A₁ bis A₃ bedeuten:
R₁ eine C₁₋₄-Alkylgruppe, eine Phenylgruppe, die mit einer C₁₋₄₋Alkylgruppe oder einem Halogenatom substituiert sein kann, das unter Chlor, Brom, Iod und Fluor ausgewählt ist;
R₂ eine C₁₋₄-Alkylgruppe oder eine Phenylgruppe,
R₃ und R₄, die gleich oder verschieden sind, eine C₁₋₄-Alkylgruppe oder eine Phenylgruppe, oder sie können in A₁ gemeinsam einen substituierten Benzolring bilden, der mit einer oder mehreren Gruppen C₁-₄-Alkyl, C₁₋₄-Alkoxy oder NO₂ substituiert ist, oder sie können in A₂ gemeinsam einen Benzolring bilden, der gegebenenfalls mit einer oder mehreren Gruppen C₁₋₄-Alkyl, C₁₋₄-Alkoxy oder NO₂ substituiert ist,
R₃ kann ferner auch ein Wasserstoffatom bedeuten,
Z ein Sauerstoffatom, ein Schwefelatom oder eine Gruppe -NR₂;
M eine Gruppe -CH, -CR (wobei R eine C₁₋₄-Alkylgruppe ist) oder -NR₅(X⁻)r;
K eine Gruppe -CH, -CR (wobei R eine C₁₋₄-Alkylgruppe ist) oder
-NR₅(X⁻)r;
P eine Gruppe -CH, -CR (wobei R eine C₁₋₄-Alkylgruppe ist) oder -NR₅(X⁻)ᵣ; wobei r Null oder 1 bedeutet;
R₅ ein Atom O⁻, eine C₁₋₄-Alkoxygruppe oder eine C₁₋₄-Alkylgruppe,
R₆ und R₇, die gleich oder verschieden sind, ein Wasserstoffatom, ein Halogenatom, das unter Chlor, Brom, Iod und Fluor ausgewählt ist, eine C₁₋₄-Alkylgruppe, eine C₁₋₄-Alkoxygruppe oder die Gruppe -NO₂;
X⁻ ein Anion, das vorzugsweise unter Chlorid, Iodid, Methylsulfat, Ethylsulfat, Acetat und Perchlorat ausgewählt ist;
mit der Maßgabe, dass
. wenn R₄ C₁₋₄-Alkyl ist und Z ein Schwefelatom bedeutet, R₃ von Wasserstoff verschieden ist;
. wenn R₅ O⁻ bedeutet, r Null ist;
. wenn K oder P oder M -N-alkyl(C₁₋₄)X⁻ bedeutet, R₆ oder R₇ von Wasserstoff verschieden ist;
. wenn K -NR₅(X⁻)r bedeutet, M = P = -CH; -CR;
. wenn M -NR₅₂(X⁻)ᵣ bedeutet, K = P = -CH; -CR;
. wenn P -NR₅₂(X⁻)ᵣ bedeutet, K = M = -CH oder -CR;
. wenn Z -NR₂ bedeutet und R₂ C₁₋₄-Alkyl ist, mindestens eine der Gruppen R₁, R₃ oder R₄ in der Struktur A₂ von C₁₋₄-Alkyl verschieden ist.
das Symbol B:
(a) eine Gruppe der folgenden Struktur B₁: 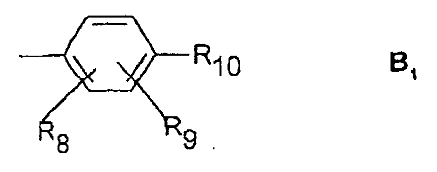 wobei in der Struktur B₁ bedeuten:
R₈ ein Wasserstoffatom, ein Halogenatom, das unter Chlor, Brom, Iod und Fluor ausgewählt ist, C₁₋₄-Alkyl, C₁₋₄-Alkoxy, -OH, -NO₂, -NHR₁₁, -NR₁₂R₁₃, -NHCOalkyl(C₁₋₄) oder R₈ bildet mit R₉ einen 5-oder 6-gliedrigen Ring, der gegebenenfalls ein oder mehrere Heteroatome enthält, die unter Stickstoff, Sauerstoff oder Schwefel ausgewählt sind;
R₉ ein Wasserstoffatom, ein Halogenatom, das unter Chlor, Brom, Iod und Fluor ausgewählt ist, C₁₋₄-Alkyl, C₁₋₄-Alkoxy oder R₉ bildet mit R₁₀ oder R₁₁ einen 5- oder 6-gliedrigen Ring, der gegebenenfalls ein oder mehrere Heteroatome enthält, die unter Stickstoff, Sauerstoff oder Schwefel ausgewählt sind;
R₁₀ Wasserstoff, -OH, -NHR₁₁ oder -NR₁₂R₁₃;
R₁₁ Wasserstoff, C₁₋₄-Alkyl, C₁₋₄-Monohydroxyalkyl, C₂₋₄-Polyhydroxyalkyl oder Phenyl;
R₁₂ und R₁₃, die gleich oder verschieden sind, C₁₋₄-Alkyl, C₁₋₄-Monohydroxyalkyl oder C₂₋₄-Polyhydroxyalkyl;
(b) eine stickstoffhaltige, 5- oder 6-gliedrige heterocyclische Gruppe, die weitere Heteroatome und/oder carbonylhaltige Gruppen enthalten und mit einer oder mehreren Gruppen C₁₋₄-Alkyl, Amino oder Phenyl substituiert sein kann, insbesondere eine Gruppe der folgenden Struktur B₂: 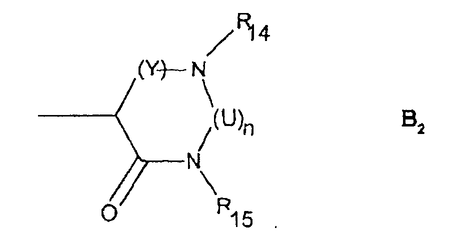 wobei in der Struktur B₂ bedeuten:
R₁₄ und R₁₅, die gleich oder verschieden sind, Wasserstoff, C₁₋₄₋Alkyl oder Phenyl;
Y die Gruppe -CO- oder die Gruppe 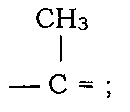
n = 0 oder 1, wobei U die Gruppe -CO- bedeutet, wenn n 1 ist;
wobei die Zusammensetzung **dadurch gekennzeichnet ist, dass** sie ferner enthält:
(ii) mindestens ein verdickendes Polymer, das unter den folgenden Polymeren ausgewählt ist:
(ii)₁ nichtionischen Guargummen;
(ii)₂ Gummen von Biopolysacchariden mikrobieller Herkunft, beispielsweise Scleroglucanen und Xanthangummi;
(ii)₃ Gummen aus pflanzlichen Exsudaten, wie Gummi arabicum, Tragant, Carrageenan, Agar und Johannisbrotkernmehl;
(ii)₄ Alginaten;
(ii)₅ Stärkeverbindungen; und
(ii)₆ Carboxyalkylcellulosen.

2. Zusammensetzung nach Anspruch 1, **dadurch gekennzeichnet, dass** es sich in der Formel (I) bei den C₁₋₄-Alkylgruppen und C₁₋₄₋Alkoxylgruppen um die Gruppen Methyl, Ethyl, Butyl, Methoxy und Ethoxy handelt.

3. Zusammensetzung nach Anspruch 2, **dadurch gekennzeichnet, dass** die kationischen Direktfarbstoffe der Formel (I) den folgenden Strukturen (I)₁ bis (I)₇₇ entsprechen:

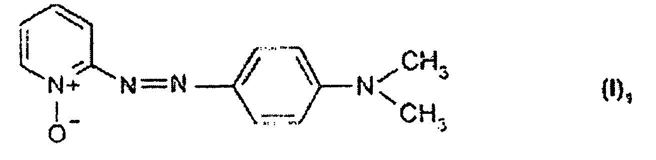

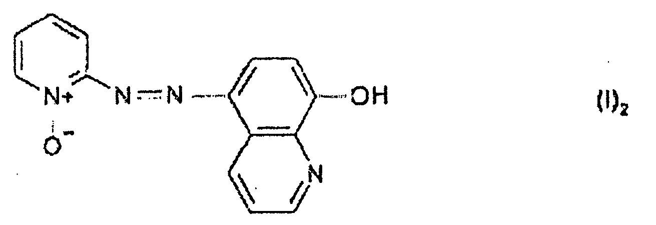

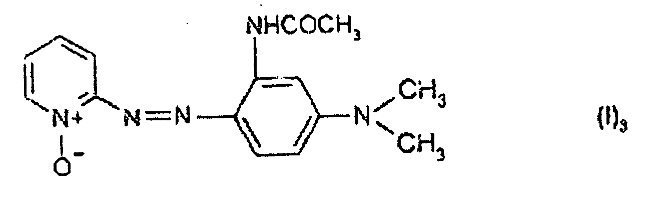

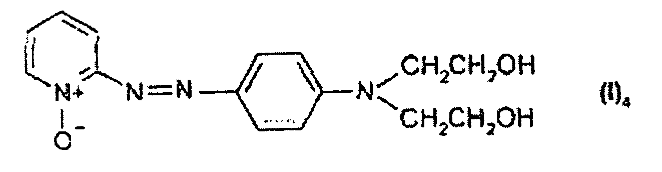

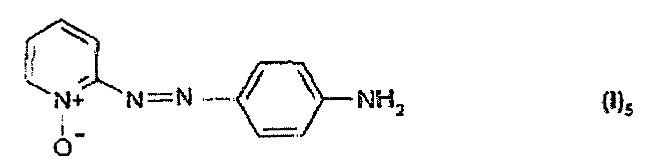

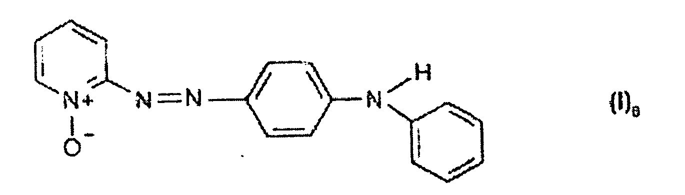

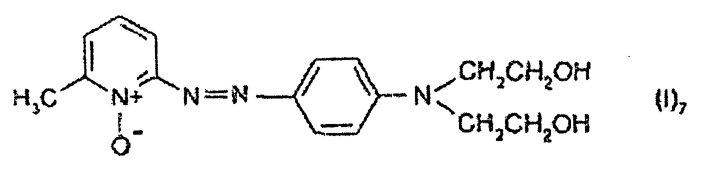

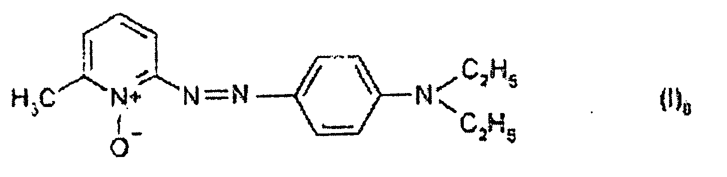

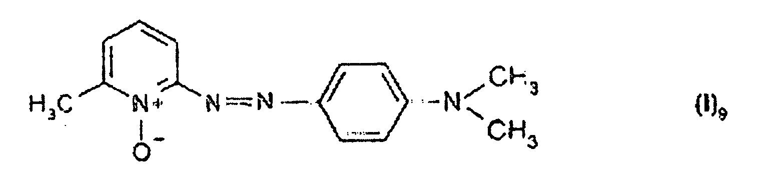

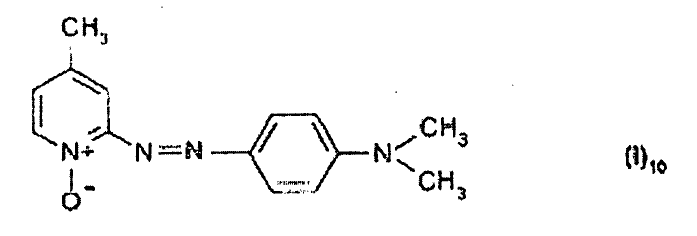

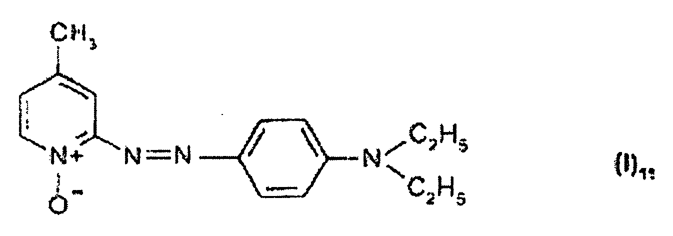

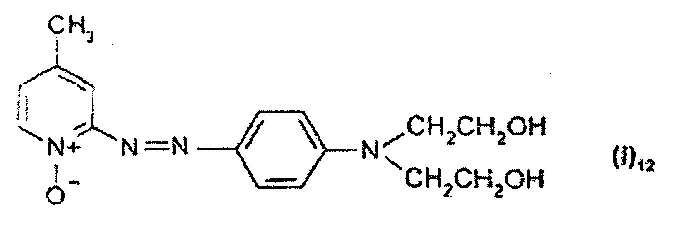

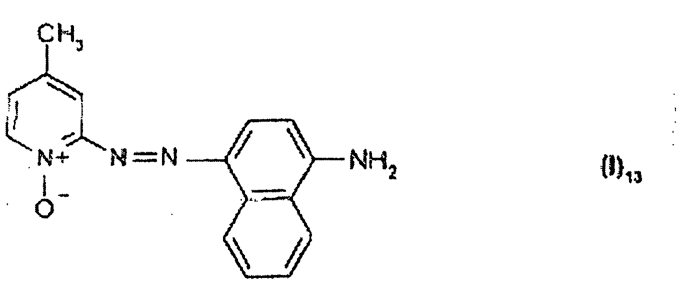

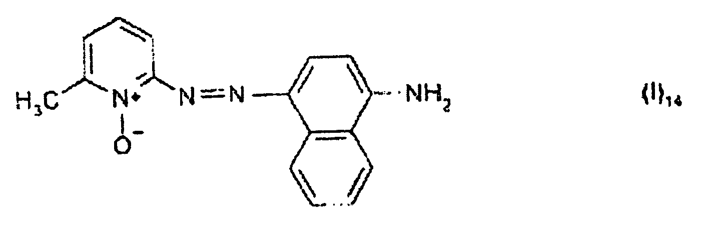

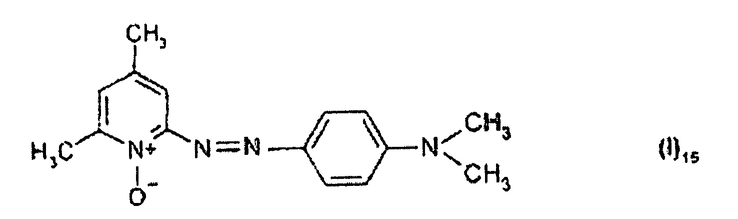

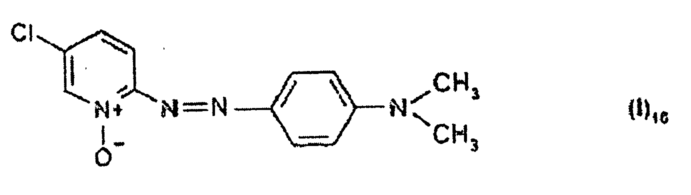

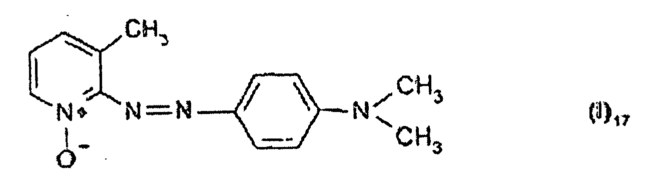

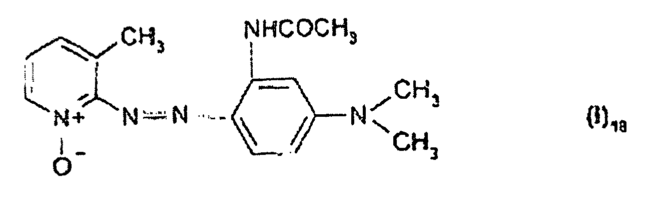

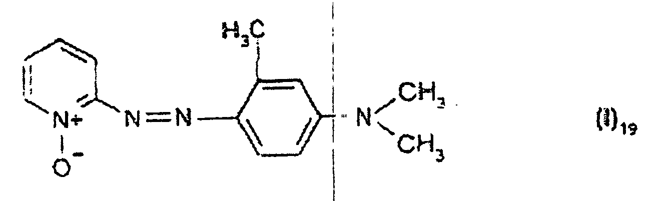

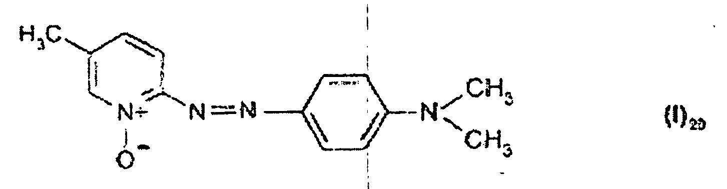

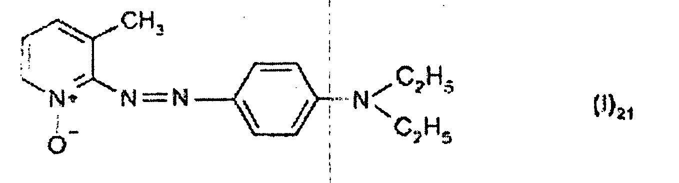

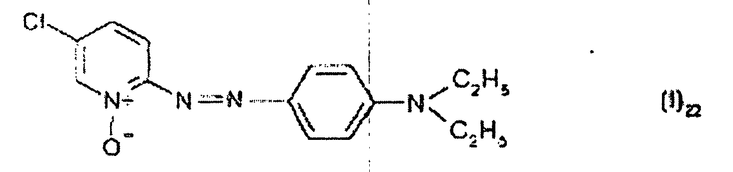

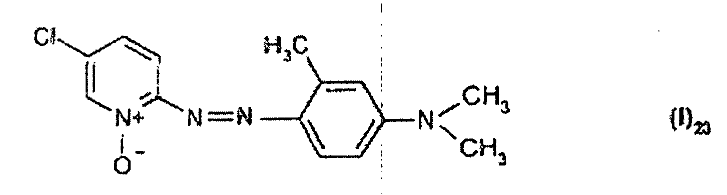

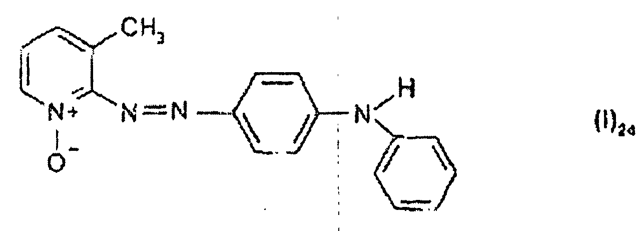

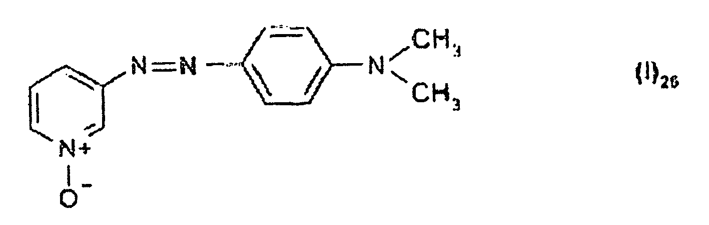

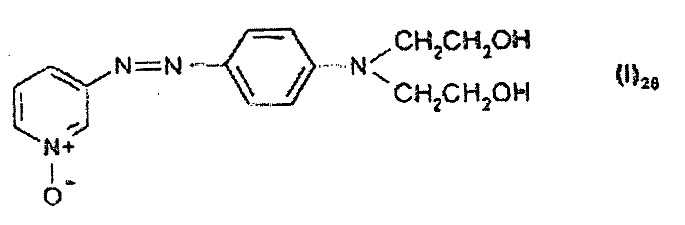

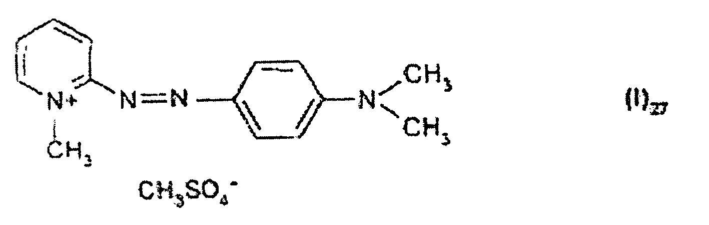

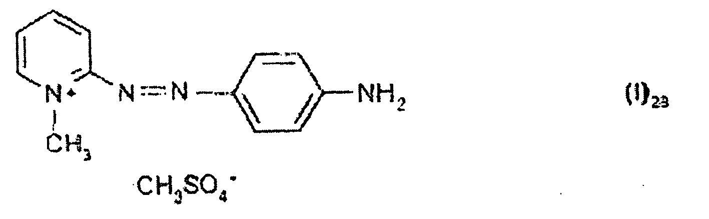

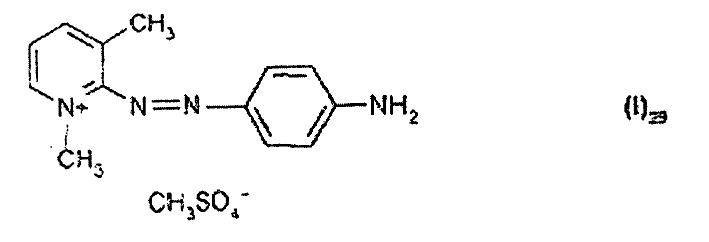

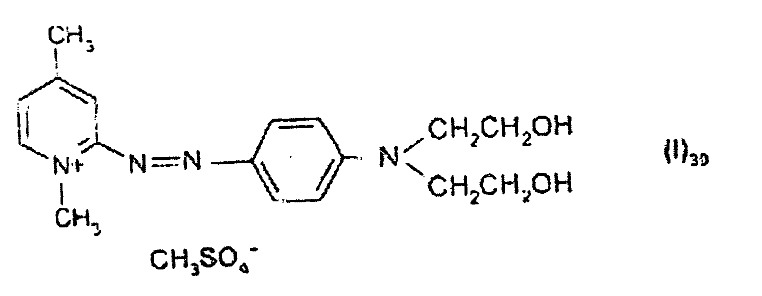

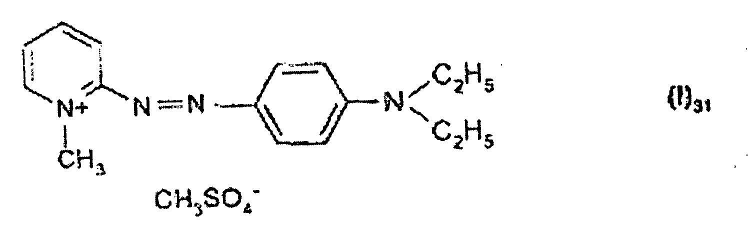

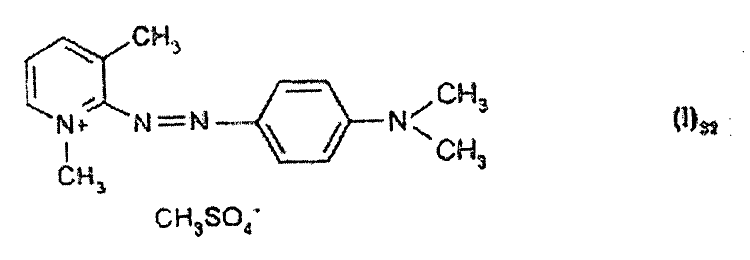

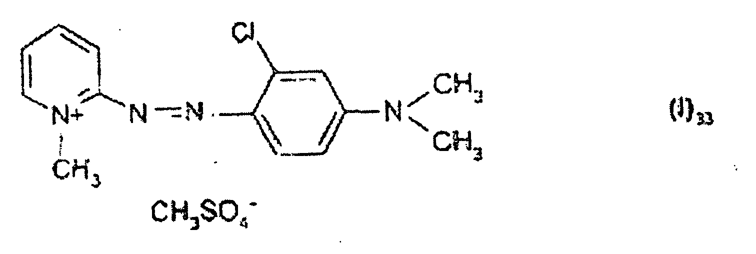

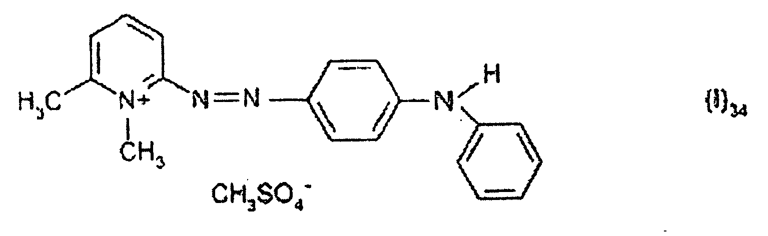

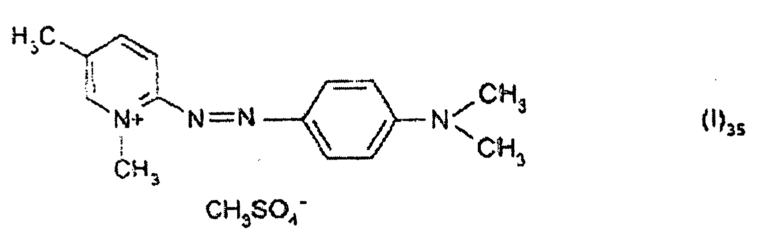

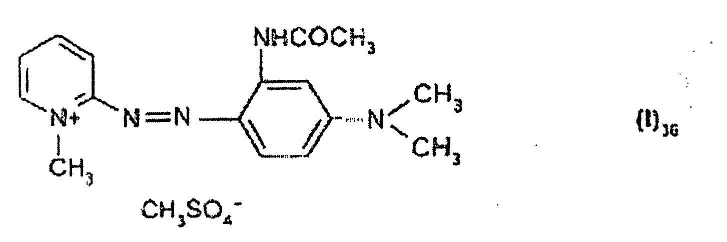

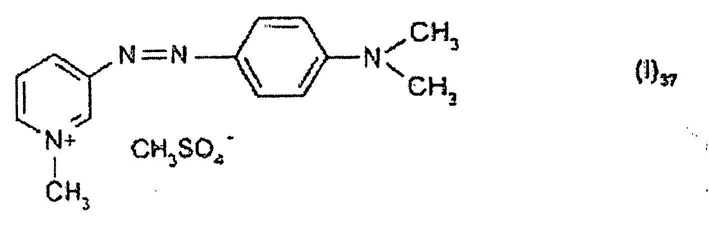

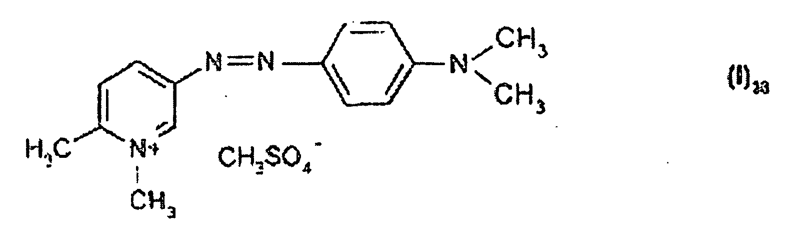

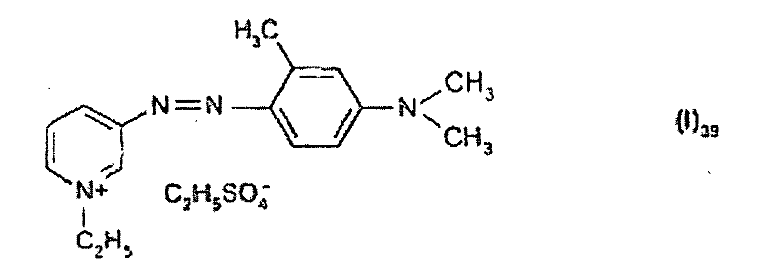

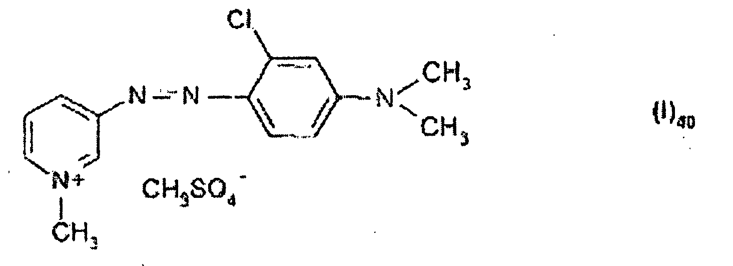

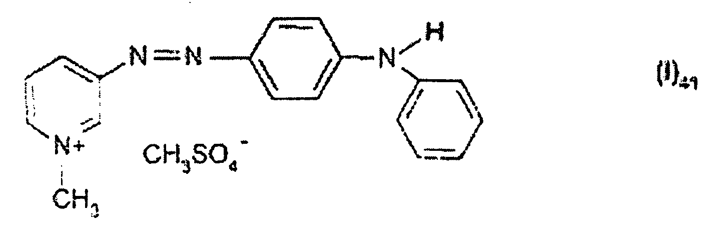

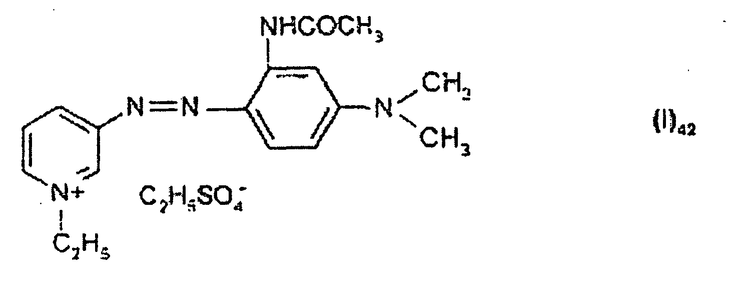

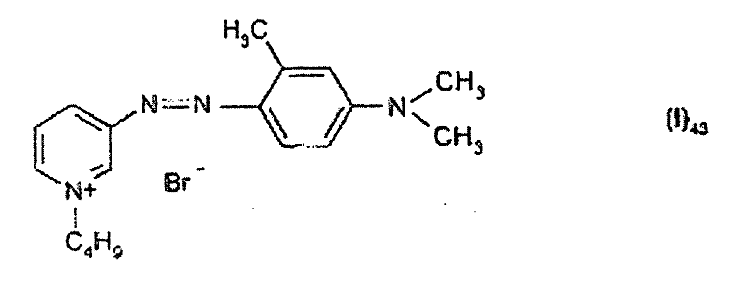

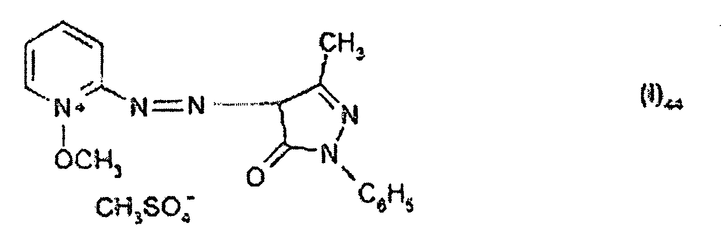

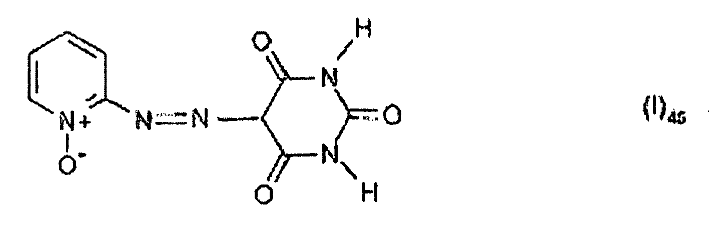

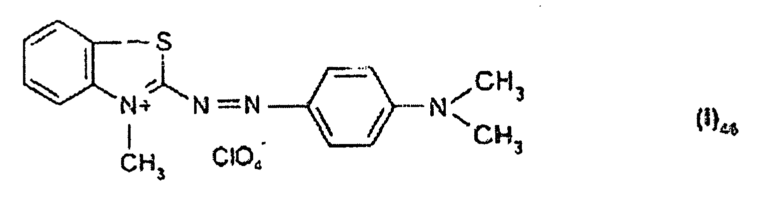

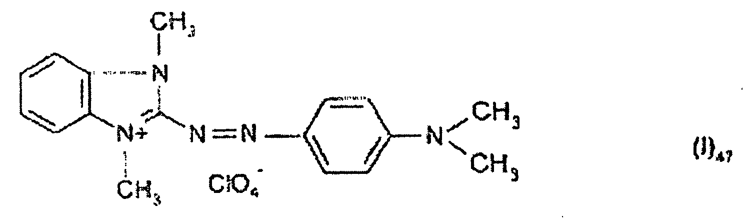

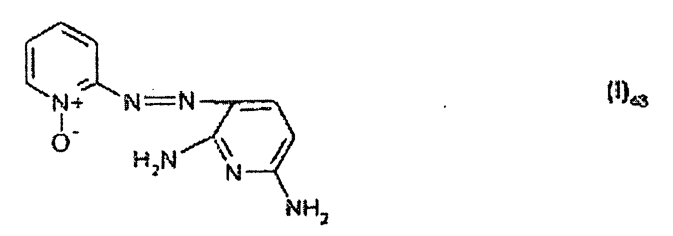

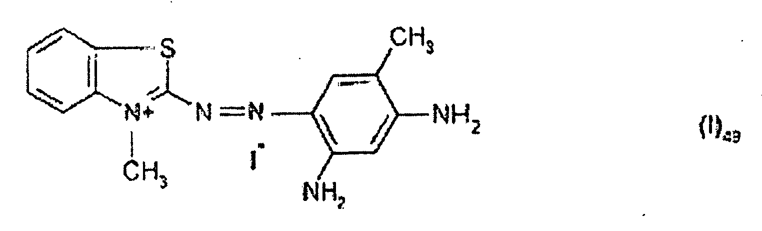

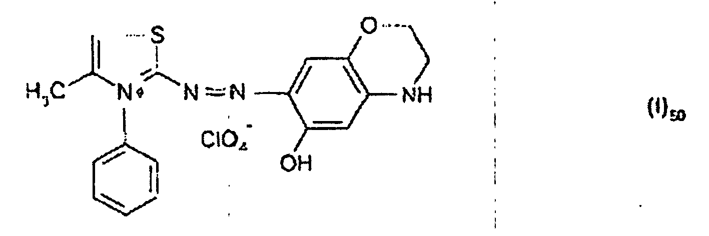

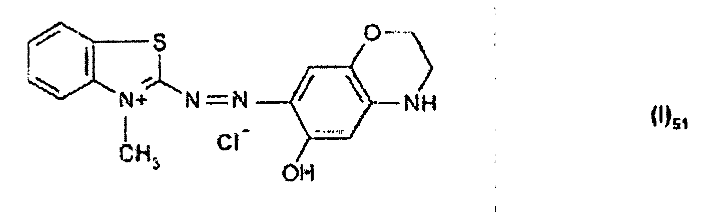

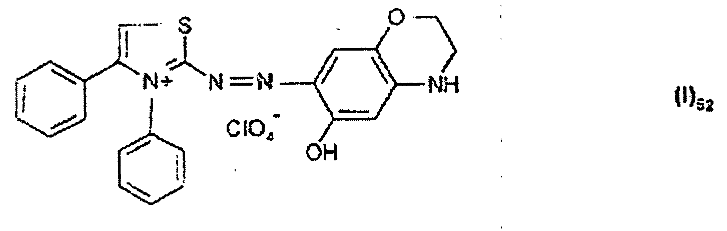

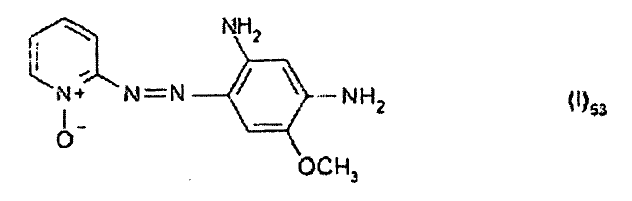

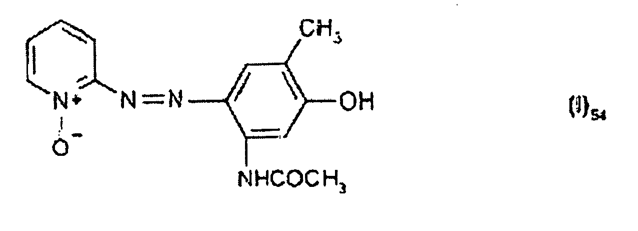

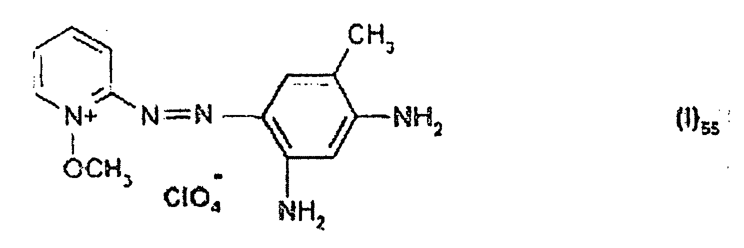

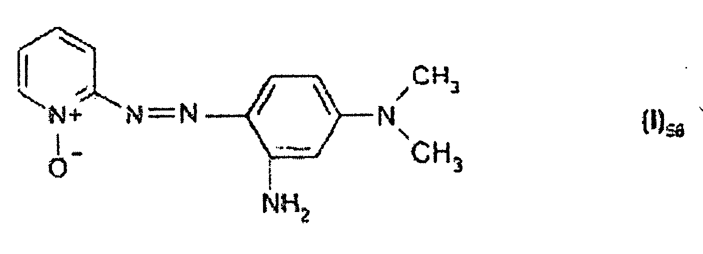

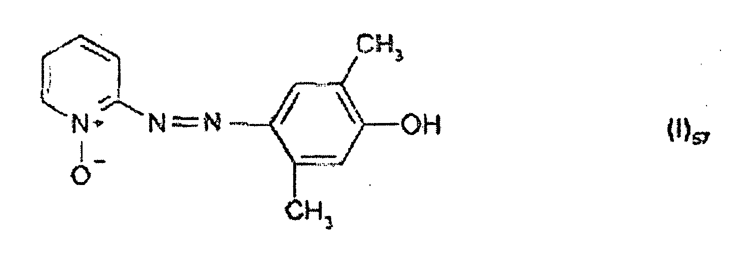

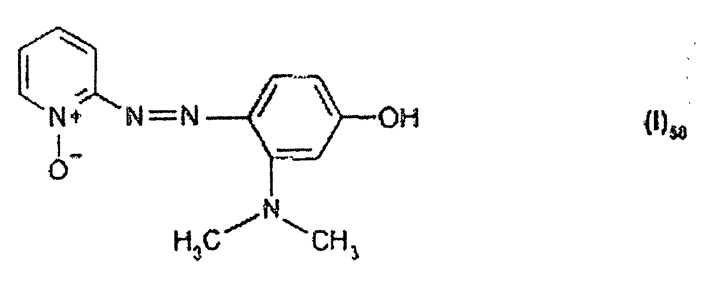

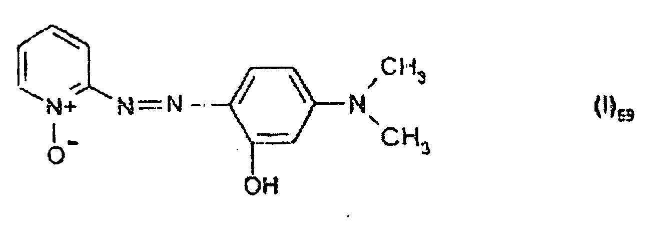

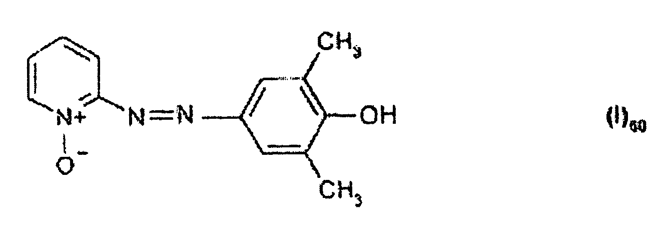

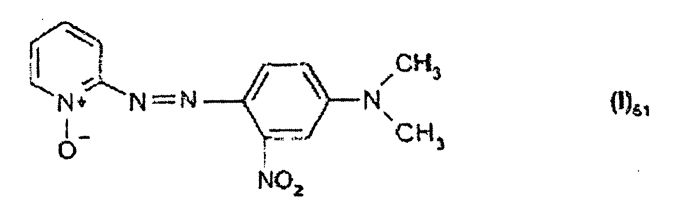

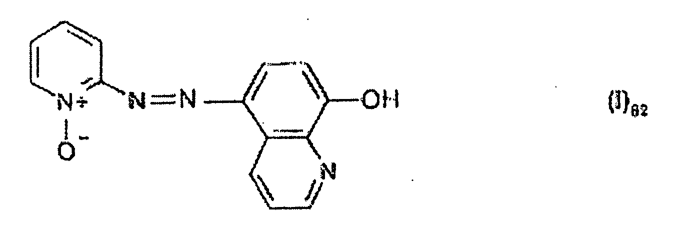

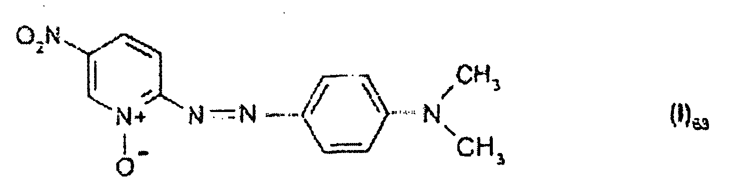

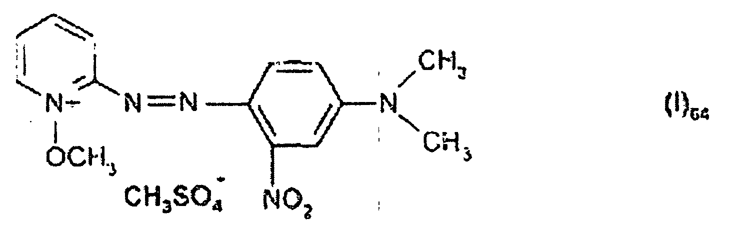

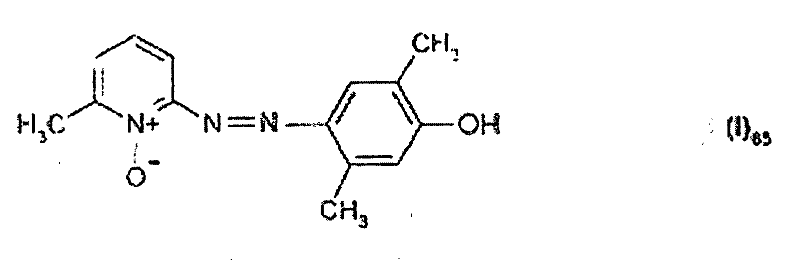

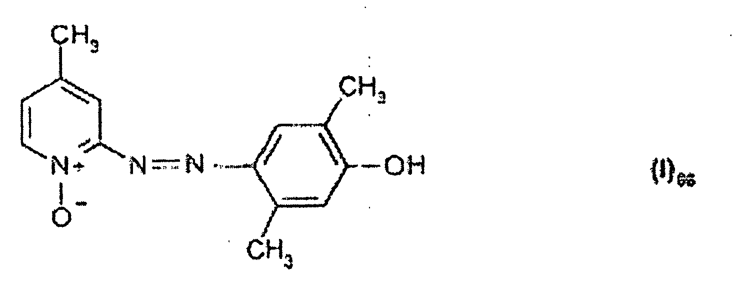

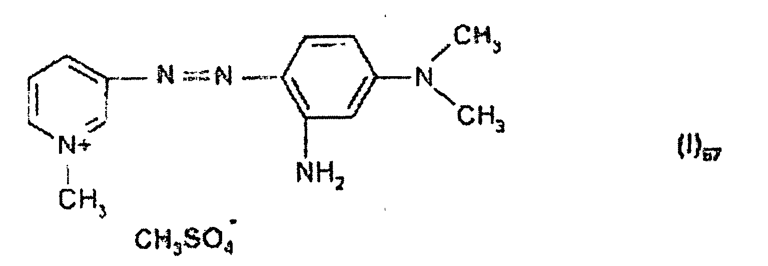

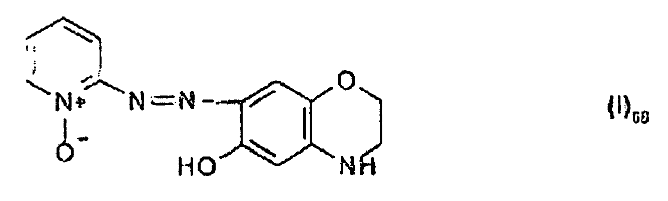

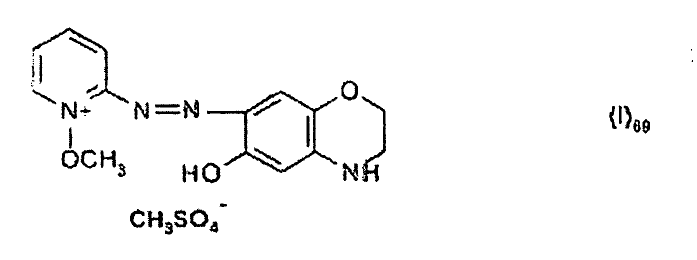

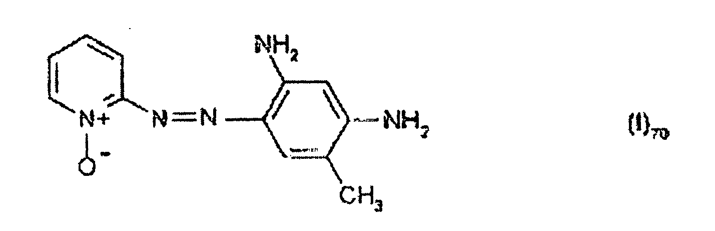

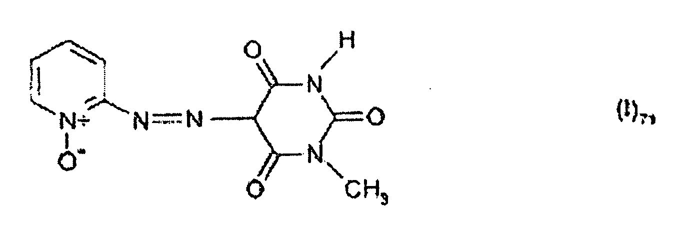

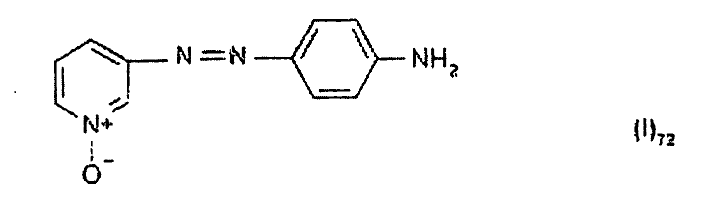

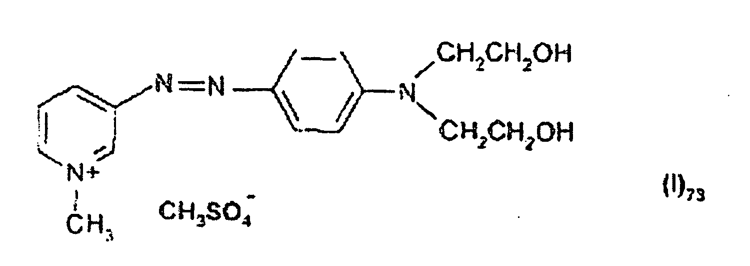

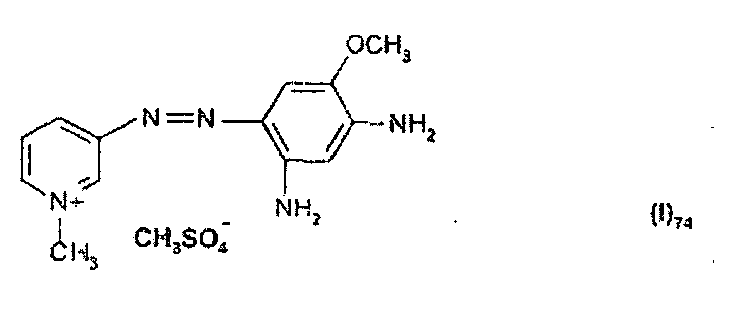

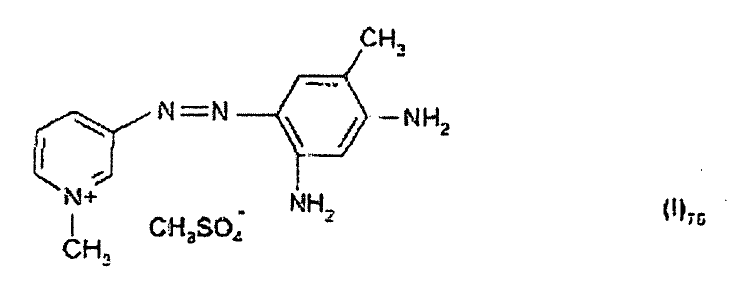

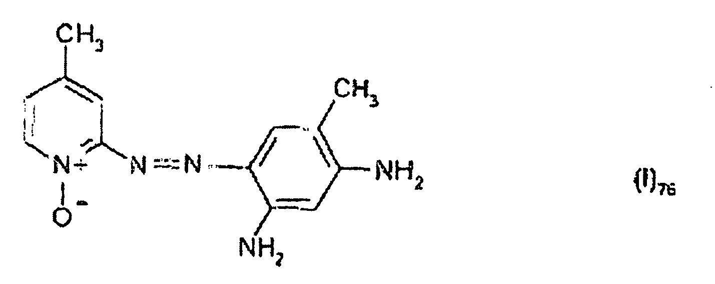

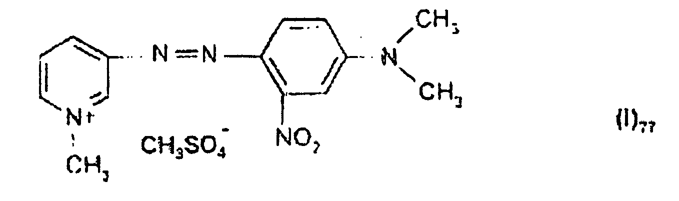

4. Zusammensetzung nach einem der vorhergehenden Ansprüche, **dadurch gekennzeichnet, dass** der oder die kationische(n) Direktfarbstoff(e) der Formel (I) 0,001 bis 10 Gew.-% des Gesamtgewichts der Zusammensetzung ausmachen.

5. Zusammensetzung nach Anspruch 4, **dadurch gekennzeichnet, dass** der oder die kationische(n) Direktfarbstoff(e) der Formel (I) 0,005 bis 5 Gew.-% des Gesamtgewichts der Zusammensetzung ausmachen.

6. Zusammensetzung nach einem der Ansprüche 1 bis 5, **dadurch gekennzeichnet, dass** das verdickende Polymer ein nichtionisches, mit C₁₋₆-Hydroxyalkylgruppen modifiziertes Guargummi ist.

7. Zusammensetzung nach Anspruch 6, **dadurch gekennzeichnet, dass** das nichtionische Guargummi einen Hydroxyalkylierungsgrad von 0,4 bis 1,2 aufweist.

8. Zusammensetzung nach einem der Ansprüche 1 bis 5, **dadurch gekennzeichnet, dass** das verdickende Polymer eine Carboxyethylcellulose ist.

9. Zusammensetzung nach einem der vorhergehenden Ansprüche, **dadurch gekennzeichnet, dass** das verdickende Polymer (ii) 0,01 bis 10 Gew.-% des Gesamtgewichts der Zusammensetzung ausmacht.

10. Zusammensetzung nach Anspruch 9, **dadurch gekennzeichnet, dass** das verdickende Polymer (ii) 0,1 bis 5 Gew.-% des Gesamtgewichts der Farbmittelzusammensetzung ausmacht.

11. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

12. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

13. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

14. Zusammensetzung nach Anspruch 13, **dadurch gekennzeichnet, dass** die Oxidationsbase(n) 0,0005 bis 12 Gew.-% des Gesamtgewichts der Farbmittelzusammensetzung ausmachen.

15. Zusammensetzung nach Anspruch 14, **dadurch gekennzeichnet, dass** die Oxidationsbase(n) 0,005 bis 6 Gew.-% des Gesamtgewichts der Farbmittelzusammensetzung ausmachen.

16. Zusammensetzung nach einem der Ansprüche 13 bis 15, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

17. Zusammensetzung nach Anspruch 16, **dadurch gekennzeichnet, dass** der oder die Kuppler 0,0001 bis 10 Gew.-% des Gesamtgewichts der Farbmittelzusammensetzung ausmachen.

18. Zusammensetzung nach Anspruch 17, **dadurch gekennzeichnet, dass** der oder die Kuppler 0,005 bis 5 Gew.-% des Gesamtgewichts der Farbmittelzusammensetzung ausmachen.

19. Zusammensetzung nach einem der vorhergehenden Ansprüche, **dadurch gekennzeichnet, dass** sie zum oxidativen Färben oder für die aufhellende Direktfärbung vorgesehen ist und mindestens ein Oxidationsmittel enthält.

20. Verfahren zum Färben von Keratinfasern und insbesondere menschlichen Keratinfasern, beispielsweise zum Färben der Haare, **dadurch gekennzeichnet, dass** auf die Fasern mindestens eine Farbmittelzusammensetzung nach einem der Ansprüche 1 bis 19 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

21. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 19 während einer Zeitspanne, die zur Bildung der gewünschten Färbung ausreichend ist, aufgebracht wird, ohne dass nochmals gespült wird.

22. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A1), die in einem zum Färben geeigneten Medium mindestens einen in den vorhergehenden Ansprüchen definierten Direktfarbstoff (i) und mindestens eine Oxidationsbase enthält, und andererseits eine Zusammensetzung (B 1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzuhg (A1) oder die Zusammensetzung (B1) das in den vorhergehenden Ansprüchen definierte verdickende Polymer (ii) enthält.

23. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A2), die in einem zum Färben geeigneten Medium mindestens einen in den vorhergehenden Ansprüchen definierten kationischen Direktfarbstoff (i) enthält, und andererseits eine Zusammensetzung (B2) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A2) oder die Zusammensetzung (B2) das in den vorhergehenden Ansprüchen definierte verdickende Polymer (ii) enthält.

24. Vorrichtung mit mehreren Abteilungen oder "Kit" zum Färben mit mehreren Abteilungen, **dadurch gekennzeichnet, dass** eine erste Abteilung die Zusammensetzung (A1) oder (A2) nach Anspruch 22 oder 23 und eine andere Abteilung die Zusammensetzung (B1) oder (B2) nach Anspruch 22 oder 23 enthält.
